# EUROPEAN PATENT SPECIFICATION

(11) **EP 2 032 161 B1**
(45) Date of publication and mention of the grant of the patent: **11.07.2012**
(21) Application number: 07730031.7
(22) Date of filing: 08.06.2007
(51) Int. Cl.: A61K 39/095

(54) **L3V LOS VACCINES**
L3V LOS IMPFSTOFFE
VACCINS L3V LOS

(30) Priority: 12.06.2006 US 804475 P; 12.06.2006 US 804489 P
(43) Date of publication of application: 11.03.2009
(73) Proprietor: GlaxoSmithKline Biologicals S.A., 1330 Rixensart (BE)
(72) Inventor: DEVOS, Nathalie, 1330 Rixensart (BE); FERON, Christiane, 1330 Rixensart (BE); POOLMAN, Jan, 1330 Rixensart (BE); WEYNANTS, Vincent, 1330 Rixensart (BE)
(74) Representative: Lubienski, Michael John
(86) International application number: PCT/EP2007/055677
(87) International publication number: WO 2007/144317

(56) References cited:
- WO-A-02/28888
- WO-A-2004/014418
- WO-A-2004/020463
- WO-A-2005/064021
- US-A1- 2006 047 106
- US-A1- 2006 073 168
- KAHLER CHARLENE M ET AL: "O-acetylation of the terminal N-acetylglucosamine of the lipooligosaccharide inner core in Neisseria meningitidis - Influence on inner core structure and assembly" JOURNAL OF BIOLOGICAL CHEMISTRY, vol. 281, no. 29, 8 May 2006 (2006-05-08), pages 19939-19948, XP002468916 ISSN: 0021-9258

## Description

### FIELD OF THE INVENTION

The present invention relates to the field of neisserial vaccine compositions, their manufacture, and the use of such compositions in medicine. More particularly it relates to processes of making novel engineered meningococcal strains which are more suitable for the production of neisserial, in particular meningococcal, outer-membrane vesicle (or bleb) vaccines. Advantageous processes and vaccine products are also described based on the use of novel LOS subunit or meningococcal outer-membrane vesicle (or bleb) vaccines which have been rendered safer and more effective for use in human subjects.

### BACKGROUND OF THE INVENTION

*Neisseria meningitidis* (meningococcus) is a Gram negative bacterium frequently isolated from the human upper respiratory tract. It is a cause of serious invasive bacterial diseases such as bacteremia and meningitis. The incidence of meningococcal disease shows geographical, seasonal and annual differences (Schwartz, B., Moore, P.S., Broome, C.V.; Clin. Microbiol. Rev. 2 (Supplement), S18-524, 1989). The bacterium is commonly classified according to the serogroup if its capsular polysaccharide.

Most disease in temperate countries is due to strains of serogroup B and varies in incidence from 1-10/100,000/year total population - sometimes reaching higher values (Kaczmarski, E.B. (1997), Commun. Dis. Rep. Rev. 7: R55-9, 1995; Scholten, R.J.P.M., Bijlmer, H.A., Poolman, J.T. et al. Clin. Infect. Dis. 16: 237-246, 1993; Cruz, C., Pavez, G., Aguilar, E., et al. Epidemiol. Infect. 105: 119-126, 1990).

Epidemics dominated by serogroup A meningococci, mostly in central Africa, sometimes reach incidence levels of up to 1000/100,000/year (Schwartz, B., Moore, P.S., Biome, C.V. Clin. Microbiol. Rev. 2 (Supplement), S18-S24, 1989). Nearly all cases as a whole of meningococcal disease are caused by serogroup A, B, C, W-135 and Y meningococci, and a tetravalent A, C, W-135, Y capsular polysaccharide vaccine is available (Armand, J., Arminjon, F., Mynard, M.C., Lafaix, C., J. Biol. Stand. 10: 335-339, 1982).

The frequency of *Neisseria meningitidis* infections has risen in the past few decades in many European countries. This has been attributed to increased transmission due to an increase in social activities (for instance swimming pools, theatres, etc.). It is no longer uncommon to isolate *Neisseria meningitidis* strains that are less sensitive or resistant to some of the standard antibiotics. This phenomenon has created an unmet medical need and demand for new anti-microbial agents, vaccines, drug screening methods, and diagnostic tests for this organism.

The available polysaccharide vaccines are currently being improved by way of chemically conjugating them to carrier proteins (Lieberman, J.M., Chiu, S.S., Wong, V.K., et al. JAMA 275 : 1499-1503, 1996).

A serogroup B vaccine, however, is not available. The serogroup B capsular polysaccharide has been found to be nonimmunogenic - most likely because it shares structural similarity with host components (Wyle, F.A., Artenstein, M.S., Brandt, M.L. et al. J. Infect. Dis. 126: 514-522, 1972; Finne, J.M., Leinonen, M., Mäkelä, P.M. Lancet ii.: 355-357, 1983). Effort has therefore been focused in trying to develop serogroup B vaccines from outer membrane vesicles (or blebs) or purified protein components therefrom.

Alternative meningococcal antigens for vaccine development are meningococcal lipooligosaccharides (LOS). These are outer membrane bound glycolipids which differ from the lipopolysaccharides (LPS) of the Enterobacteriaceae by lacking the O side chains, and thus resemble the rough form of LPS (Griffiss et al. Rev Infect Dis 1988; 10: S287-295). Heterogeneity within the oligosaccharide moiety of the LOS generates structural and antigenic diversity among different meningococcal strains (Griffiss et al. Inf. Immun. 1987; 55: 1792-1800). This has been used to subdivide the strains into 12 immunotypes (Scholtan et al. J Med Microbiol 1994, 41:236-243). Immunotyping is usually carried out be the Ouchterlony method using adsorbed polyclonal antibodies generated against LOS of known immunotype (Poolman JT, Hopman CTP and Zanen HC, FEMS Microbiol Letters (1982) 13: 339-348). Immunotypes L3, L7, & L9 are immunologically identical and are structurally similar (or even the same) and have therefore been designated L3,7,9 (or, for the purposes of this specification, generically as "L3"). Meningococcal LOS L3,7,9 (L3), L2 and L5 can be modified by sialylation, or by the addition of cytidine 5'-monophosphate-N-acetylneuraminic acid. Although L2, L4 and L6 LOS are distinguishable immunologically, they are structurally similar and where L2 is mentioned herein, either L4 or L6 may be optionally substituted within the scope of the invention. Antibodies to LOS have been shown to protect in experimental rats against infection and to contribute to the bactericidal activity in children infected with *N. meningitidis* (Griffiss et al J Infect Dis 1984; 150: 71-79).

A problem associated with the use of LOS in a meningococcal vaccine, however, is its toxicity (due to its Lipid A moiety).

LOS is also present on the surface of meningococcal blebs. For many years efforts have been focused on developing meningococcal outer membrane vesicle (or bleb) based vaccines (de Moraes, J.C., Perkins, B., Camargo, M.C. et al. Lancet 340: 1074-1078, 1992; Bjune, G., Hoiby, E.A. Gronnesby, J.K. et al. 338: 1093-1096, 1991). Such vaccines have the advantage of including several integral outer-membrane proteins in a properly folded conformation which can elicit a protective immunological response when administered to a host. In addition, Neisserial strains (including *N. meningitidis* serogroup B - menB) excrete outer membrane blebs in sufficient quantities to allow their manufacture on an industrial scale. More often, however, blebs are prepared by methods comprising a 0.5% detergent (e.g. deoxycholate) extraction of the bacterial cells (e.g. EP 11243). Although this is desired due to the toxicity of LOS (also called endotoxin) as described above, it also has the effect removing most of the LOS antigen from the vaccine.
A further problem with using LOS as a vaccine antigen is that 12 LPS immunotypes exist with a diverse range of carbohydrate-structures (M. P. Jennings et al, Microbiology 1999, 145, 3013-3021; Mol Microbiol 2002, 43:931-43). Antibodies raised against one immunotype fail to recognise a different immunotype. Although effort has been focused on producing a generic "core" region of the oligosaccharide portions of the LOS immunotypes (e.g. WO 94/08021), the bactericidal activity of antibodies generated against the modified LOS is lost. Thus a vaccine may need to have many LOS components of different immunotype to be effective.
A further problem exists with the use of LOS (also known as LPS or lipopolysaccharide) as antigens in human vaccines, namely that they carry saccharide structures that are similar to human saccharide structures (for instance on human red blood cells), thus posing a safety issue with their use. Yet changing the LOS structure is problematic due to the structural sensitivity of the bactericidal effectiveness of the LOS antigen.

WO 2004/014417 describes certain solutions to these problems.

The present inventors have found furthermore that:
- the decoration of the LOS inner core is important in defining the bactericidal epitope of the immunotype,
- it has been shown that combinations of LOS of differing structures can be effective against meningococcal disease of sergroups A, B, C, W135 and Y,
- a known variant immunotype L3V is not killed by anti-L3 sera, but by anti-L2 sera,
- the enzyme by which L2 LOS undergoes the O-acetylation decoration on the GlcNAc residue attached to Heptose II has been found and the gene encoding it - Oac 1,
- L3 immunotype LOS has been found which is O-acetylated (never previously reported), and this seems to be widespread amongst L3 strains,
- although antibodies against conventional L3 LOS can kill O-acetylated L3 strains, killing is not as efficient as against conventional L3 strains.

The above findings have lead the inventors towards suggesting the use of neisserial LOS in new and beneficial ways in the manufacture of Neisserial vaccine formulations.

### SUMMARY OF THE INVENTION

Accordingly the present invention provides a Vaccine comprising L3V LOS and L3 LOS from Neisserial strains, wherein the LOS has the following structure: 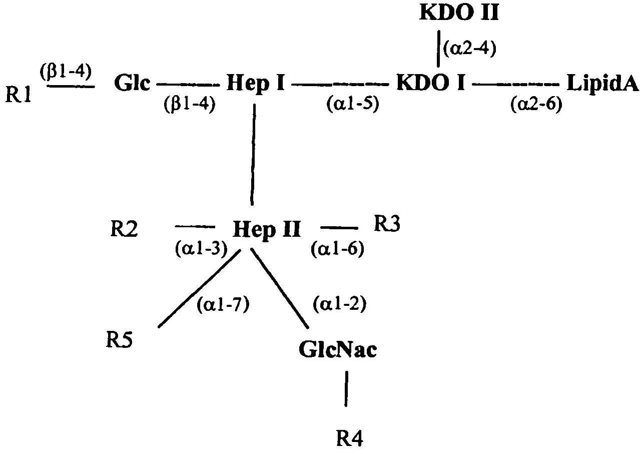 and wherein for the L3V LOS: 
R1= GlcNac — Gal — , Gal — GlcNac — Gal — , or NeuNac — Gal — GlcNac — Gal — ,
(β1-3) (β1-4) (β1-3) (α2-3) (β1-4) (β1-3)
R2= PEA, R3= PEA,
R4= H or OAc,
R5=H, PEA, or Gly, and wherein for the L3 LOS: 
R1= GlcNac — Gal — , Gal — GlcNac — Gal — ,or NeuNac — Gal — GlcNac — Gal —
(β1-3) (β1-4) (β1-3) (α2-3) (β1-4) (β1-3)
R2= PEA,
R3= H,
R4= H or OAc,
R5=H, orGly further comprising a pharmaceutically acceptable excipient.

The terms above refer to standard abbreviations in the LOS art, for instance Glc refers to Glucose (or D-glucopyranose), KDO refers to 2-keto-3-deoxyoctonate, Hep refers to L-glycero-D-manno-heptose, GlcNAc to N-acetylglucosamine, Gal to Galactose, NeuNac to sialic acid, OAc to O-acetyl, PEA to phosphethanolamine (or 2-aminoethyl phosphate), Gly to Glycine, etc.

Every instance of "neisserial" in this specification can indicate *N. meningitidis,* for instance serogroups A, B, C, W135 and Y. It may also indicate any other strain (such as gonococcus or Neisseria lactamica) that may produce the LOS of the invention.

The L2, L3V or L3 LOS of the invention may be isolated from a *Neisseria meningitidis* A, B, C, W135 or Y strain.

In a further aspect of the invention the vaccine or immunogenic composition of the invention may further comprise L10 LOS from a Neisserial strain, optionally isolated from a *Neisseria meningitidis* A, B, C, W135 or Y strain.

For instance, the vaccine or immunogenic composition of the invention may further comprise L10 LOS with the following structure: 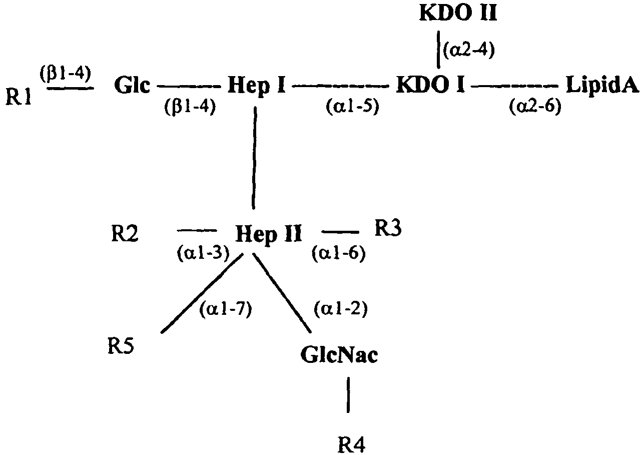 wherein:
R1=Hexose-Hexose-
R2= H or PEA,
R3= PEA,
R4= OAc,
R5=H or Gly.
"R1=Hexose-Hexose-" may cover Gal-Gal- , Gal-Glc- , Glc-Gal- , or Glc-Glc-residues, for instance:

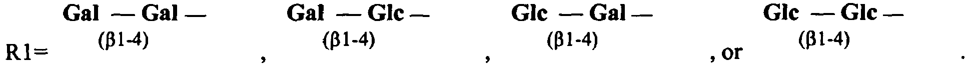

The R1 terminal Hexose in the L10 LOS of the invention may or may not be sialylated (if so then optionally through an α-Neu5Ac-(2→3) group).

Where the L3V LOS of the vaccine or immunogenic composition of the invention has R2=PEA the immunogenic composition of the invention may further comprise L4 LOS of the invention of the following structure: 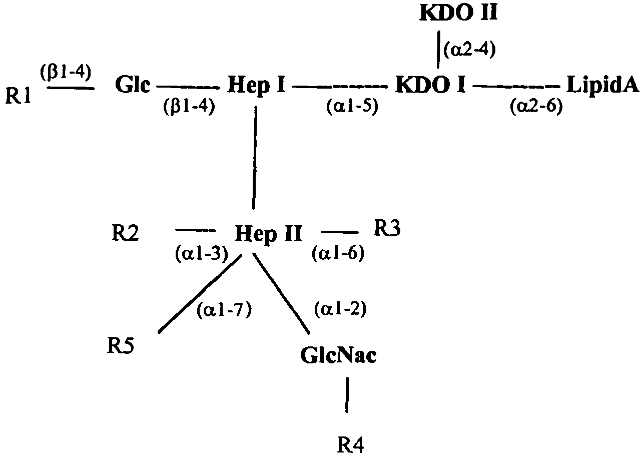 wherein for the L4 LOS: 
R1= GlcNac — Gal — , Gal — GlcNac — Gal — ,or NeuNac — Gal — GlcNac — Gal — ,
(β1-3) (β1-4) (β1-3) (α2-3) (β1-4) (β1-3)
R2= H,
R3= PEA,
R4= OAc,
R5=Gly.

Alternatively the L4 LOS of the invention may have R4=H and/or R5=H.

The L4 LOS of the invention may be isolated from a *Neisseria meningitidis* A, B, C, W135 or Y strain.

By "one or more of the LOS of the invention" or "LOS of the invention" or similar phrases herein it is meant L2 LOS, L3V LOS, L3 LOS L10 LOS, L4 LOS, L2 or L3V and L3 LOS, L2 or L3V and L10 LOS, L3 and L10 LOS, L4 and L2 or L3V LOS, L4 and L3 LOS, L4 and L10 LOS, L2 or L3V and L3 and L10 LOS, L2 or L3V and L3 and L4 LOS, L2 or L3V and L4 and L10 LOS, L3 and L4 and L10 LOS, or L2 or L3V and L3 and L10 and L4 LOS of the invention. By "bleb preparations of the invention" or similar phrases herein it is meant one or more blebs of the invention that have L2 LOS, L3V LOS, L3 LOS, L10 LOS, L4 LOS, L2 or L3V and L3 LOS, L2 or L3V and L10 LOS, L3 and L10 LOS, L4 and L2 or L3V LOS, L4 and L3 LOS, L4 and L10 LOS, L2 or L3V and L3 and L10 LOS, L2 or L3V and L3 and L4 LOS, L2 or L3V and L4 and L10 LOS, L3 and L4 and L10 LOS, or L2 or L3V and L3 and L10 and L4 LOS of the invention. Bacterial strains of the invention are those from which one or more of the LOS of the invention may be isolated.

One or more of the LOS of the invention may be conjugated to a protein carrier (a source of T helper epitopes - such as tetanus toxoid, Diphtheria toxoid, CRM197, or an outer membrane protein on a meningococcal) bleb (see below). One or more may have its LipidA moiety detoxified either chemically (see below) or genetically (for instance if isolated from a neisserial strain which is msbB(-) and/or htrB(-) - see below).

One or more LOS of the invention may be present in the immunogenic composition or vaccine as a purified LOS preparation, as a liposomal preparation (typically comprising purified LOS), or as a bleb preparation (see below).

Bleb preparation(s) of the invention may be isolated from their respective neisserial strains after an extraction step using 0-0.5, 0.02-0.4, 0.04-0.3, 0.06-0.2, 0.08-0.15 or 0.09-0.11 % detergent, preferably deoxycholate. Their respective neisserial strains may have been cultured in conditions with iron available to it, or in conditions of iron depletion (e.g. with added iron chelator such as desferral - see below). The bleb preparations of the invention may be isolated from a neisserial strain which cannot synthesise capsular polysaccharide. For instance the strain may have one of the following capsular polysaccharide genes downregulated in expression, or deleted (i.e. no functional expression from the gene), compared to the native strain from which it is derived: ctrA, ctrB, ctrC, ctrD, synA, synB, synC, or, preferably, siaD. Where L2 or L3V and L3 blebs are both present (or more than one bleb preparation of the invention is present), the strains from which they are derived may have the same capsular polysaccharide gene downregulated in expression in each strain. The neisserial strain may have either or both of the following lipid A genes downregulated in expression, and preferably deleted (i.e. no functional expression from the gene), compared to the native strain from which it is derived: msbB or htrB, preferably the former. Where L2 or L3V and L3 blebs are both present (or more than one bleb preparation of the invention is present), the strains from which the blebs are derived preferably have the same lipid A gene(s) downregulated in expression in each strain. The neisserial strain may have 1 or more of the following outer membrane protein genes downregulated in expression, and preferably deleted (i.e. no expression of the gene product on the outer membrane of the strain), compared to the native strain from which it is derived: porA, porB, opA, opC, pilC, lbpA or frpB. Where L2 or L3V and L3 blebs are both present (or more than one bleb preparation of the invention is present), the strains preferably have the same outer membrane protein gene(s) downregulated in expression in each neisserial strain. The neisserial strain may have 1 or more of the following outer membrane protein antigens upregulated in expression: NspA, TbpA low, TbpA high, Hsf, Hap, OMP85, PilQ, NadA, GNA1870, MltA. Where L2 or L3V and L3 blebs are both present (or more than one bleb preparation of the invention is present), the strains from which they are derived preferably have one or more different outer membrane protein antigens upregulated in expression in each strain.

Vaccine compositions comprising an effective amount of the immunogenic composition of the invention and a pharmaceutically acceptable carrier are further provided. The vaccine may additionally comprise an adjuvant, for example aluminium hydroxide or aluminium phosphate. The vaccine may additionally comprise one or more conjugated capsular polysaccharides or oligosaccharides derived from the following strains: meningococcus serogroup A, meningococcus serogroup C, meningococcus serogroup W-135, meningococcus serogroup Y, and *H. influenzae* type b.

A use of the immunogenic composition of the invention or the vaccine of the invention in the manufacture of a medicament for the prevention or treatment of disease caused by one or more *N. meningitidis* serogroups selected from the following list: A, B, C, W135, and Y is also provided. Further provided is a method of prevention or treatment of disease caused by one or more *N. meningitidis* serogroups selected from the following list: A, B, C, W135, and Y, comprising the step of administering the immunogenic composition of the invention or the vaccine of the invention to a human patient in need thereof.

Furthermore there is provided a process of manufacturing the vaccines of the invention comprising the step of isolating the or L3V LOS and the L3 LOS, combining the L3V and L3 LOS, and formulating the L3V and L3 LOS with a pharmaceutically acceptable excipient.

The inventors have also found that strains of L3V immunotype are surprisingly susceptible to being killed by sera generated against L2 immunotype LOS. For the purposes of this invention the L3V immunotype is not classed an L3 strain as it is immunologically more similar to the L2 immunotype.

Accordingly in a further aspect of the invention there is provided a use of an immunogenic composition comprising L3V LOS from a Neisserial strain in the manufacture of a medicament for the prevention or treatment of *N. meningitidis* immunotype L2 disease, or of an immunogenic composition comprising L2 LOS from a Neisserial strain in the manufacture of a medicament for the prevention or treatment of *N. meningitidis* immunotype L3V disease wherein the L3V LOS has the following structure: 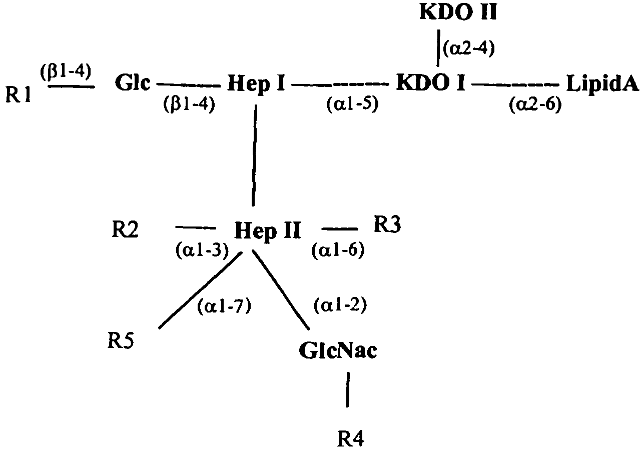 wherein: 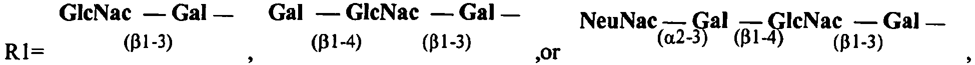
R2= PEA,
R3= PEA,
R4= H or OAc,
R5=H, PEA, or Gly.

There is also provided a method of preventing or treating *N. meningitidis* immunotype L2 disease comprising the step of administering to a human patient in need thereof an effective amount of an immunogenic composition comprising L3V LOS from a Neisserial strain, or of preventing or treating *N. meningitidis* immunotype L3V disease comprising the step of administering to a human patient in need thereof an effective amount of an immunogenic composition comprising L2 LOS from a Neisserial strain.

The above may also be the structure of the LOS carried by the disease causing N. *meningitidis* immunotype L3V referred to above for this aspect of the invention.

In the above use/method of this aspect of the invention the L2 LOS may have the following structure: 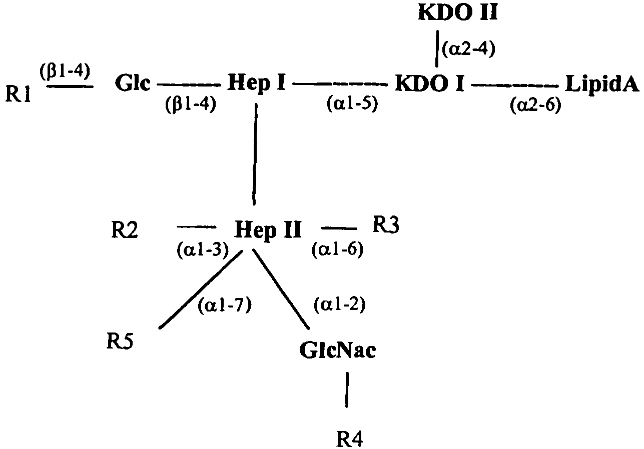 wherein: 
GlcNac — Gal —    Gal — GlcNac — Gal —
NeuNac — Gal — GlcNac — Gal —
(β1-3)    (β1-4)    (β1-3)    (α2-3)    (β1-4)
R1=    ,    ,or    (β1-3)    ,
R2= Glc,
R3= PEA,
R4= H or OAc,
R5=H, PEA, or Gly,

The above may also be the structure of the LOS carried by the disease causing N. meningitidis immunotype L2 referred to above for this aspect of the invention.

As explained throughout this specification for LOS of the present invention, the L2 or L3V LOS of this aspect of the invention may be conjugated to a protein carrier (see above and below for further explanation), it may comprise a detoxified lipid A moiety, for instance lacking a secondary acyl chain consistent with the LOS having been isolated from a msbB(-) neisserial strain (and/or through the LOS being complexed to the Lipid A detoxifying peptides described below), it may be present in the immunogenic composition as a purified LOS preparation, as a liposomal preparation, or as a bleb preparation. If a bleb preparation it may be isolated from its respective neisserial strain after an extraction step using 0-0.5, 0.02-0.4, 0.04-0.3, 0.06-0.2, 0.08-0.15 or 0.09-0.11 % detergent, preferably deoxycholate. The neisserial strain may not be able to synthesise capsular polysaccharide, for instance it may have one of the following capsular polysaccharide genes downregulated in expression, and preferably deleted (no functional expression), compared to the native strain from which it is derived: ctrA, ctrB, ctrC, ctrD, synA, synB, synC, or, preferably, siaD. The neisserial strain may have either or both of the following lipid A genes downregulated in expression, and preferably deleted (no functional expression), compared to the native strain from which it is derived: msbB or htrB, preferably the former.

### FIGURE LEGENDS

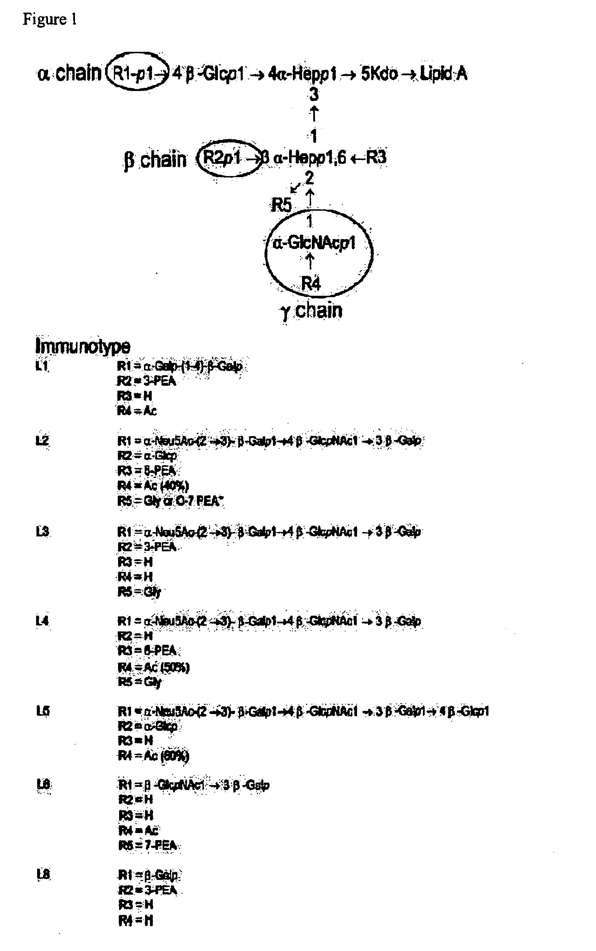

Figure 1: The common substitution patterns of meningococcal LOS structures as demonstrated by structures of L1-L8 immunotypes (taken from Kahler et al. Glycobiology 2005 15:409-419 / 2006 JBC 281:19939-19948). The L7 immunotype structure (not shown) is the non-sialylated version of the L3 immunotype structure. The conserved inner core region is shown with variable attachments denoted as R1-R5. The composition of the α-chain (R1) is governed by the phase-variable expression of the *lgtA-E* transferases and *lst,* which encodes the sialyltransferase that attaches the terminal α -Neu5Ac (sialic acid) group. Note that attachment of glycine to the inner cores is via position 7 on the second Hep residue. Note a further KDO residue is not shown which is known to be present in the inner-core for all immunotypes attached to the KDO residue shown in the diagram.
Figure 2A: Schematic of the LOS structures of various L3 *N. meningitidis* strains as determined by mass spectrometry. Interestingly, some L3 strains less prone to being killed by H44/76 derived sera are O-acetylated.
Figure 2B: Common LOS structures of various *N. meningitidis* immunotypes. Names of known genes encoding enzymes for forming certain parts of the LOS structure are given.
Figure
   3A: *N. meningitidis* LOS O-acetylation gene NMA 2202 from strain Z2491. Note sequence of 5 Gs in the open reading frame (upper case) renders the open reading frame in frame.
   3B: *N. meningitidis* LOS O-acetylation gene NMB 0285 from strain MC58. Open reading frame in upper case, surrounding sequences (e.g. promoter sequence) in lower case. Note sequence of 6 Gs in the open reading frame (underlined) renders the open reading frame out of frame.
   3C: *N. meningitidis* LOS O-acetylation gene (NMB 0285 equivalent) from strain 760676. Open reading frame in upper case, surrounding sequences (e.g. promoter sequence) in lower case. Note sequence of 5 Gs in the open reading frame (underlined) renders the open reading frame in frame.
   3D: *N. meningitidis* LOS O-acetylation gene (NMB 0285 equivalent) from (MenB, L3) strain NZ124. Open reading frame in upper case, surrounding sequences (e.g. promoter sequence) in lower case. Note sequence of 5 Gs in the open reading frame (underlined) renders the open reading frame in frame.

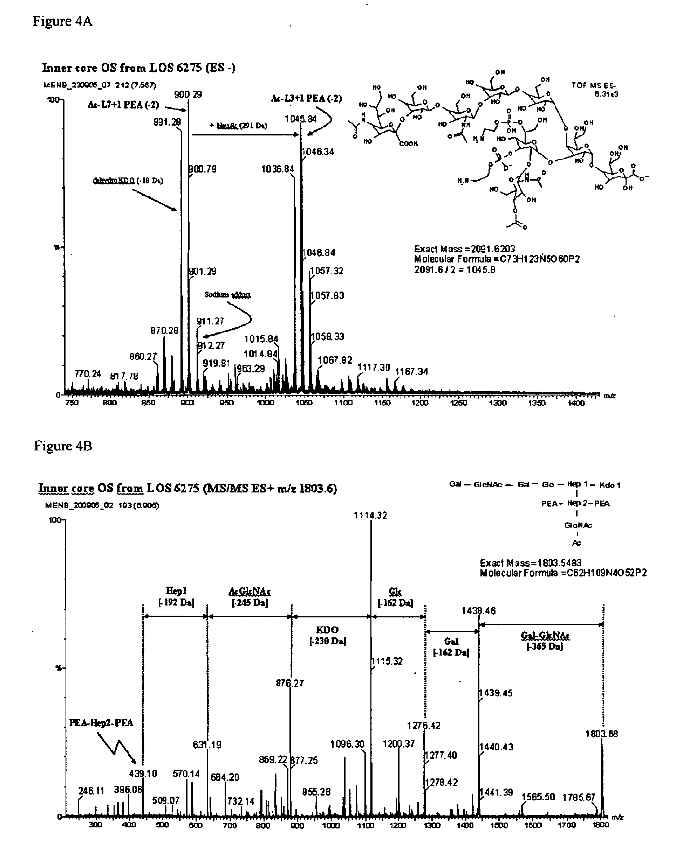

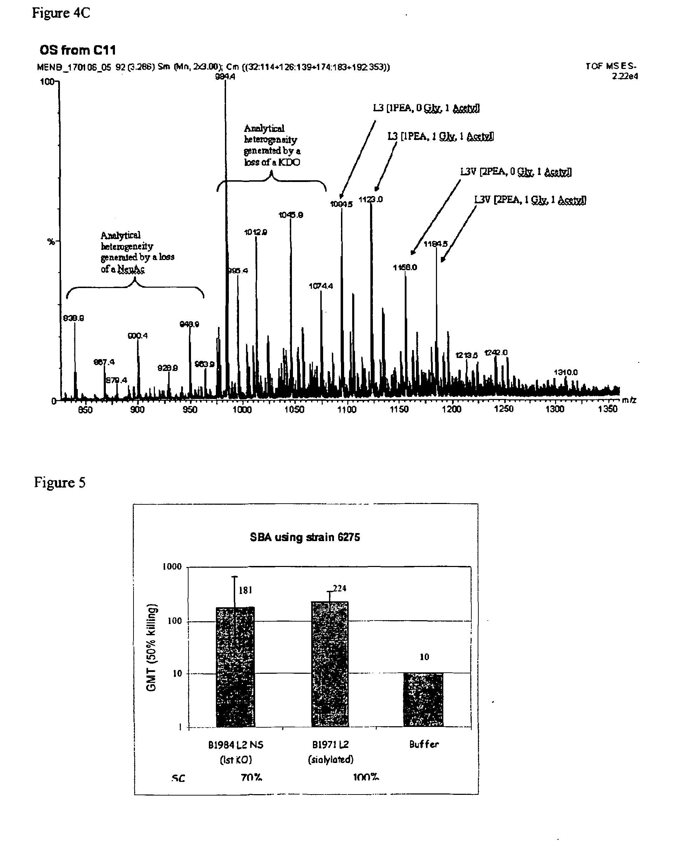

Figure 4: A - Inner core oligosaccharide from LOS 6275 (ES-); B - Inner core oligosaccharide from LOS 6275 (MS/MS ES+ m/z 1803.6), schematic shown of the structure showing HepII linked to PEA at positions 3 and 6, and inner core being O-acetylated; C - Inner core oligosaccharide from Men C strain C11 (ES-).
Figure 5: Impact of sialylation of L2 LOS on the induction of cross-bactericidal antibodies by L2 derived OMV vaccines. SBA titers (GMT for 50% killing) and seroconversion (%).

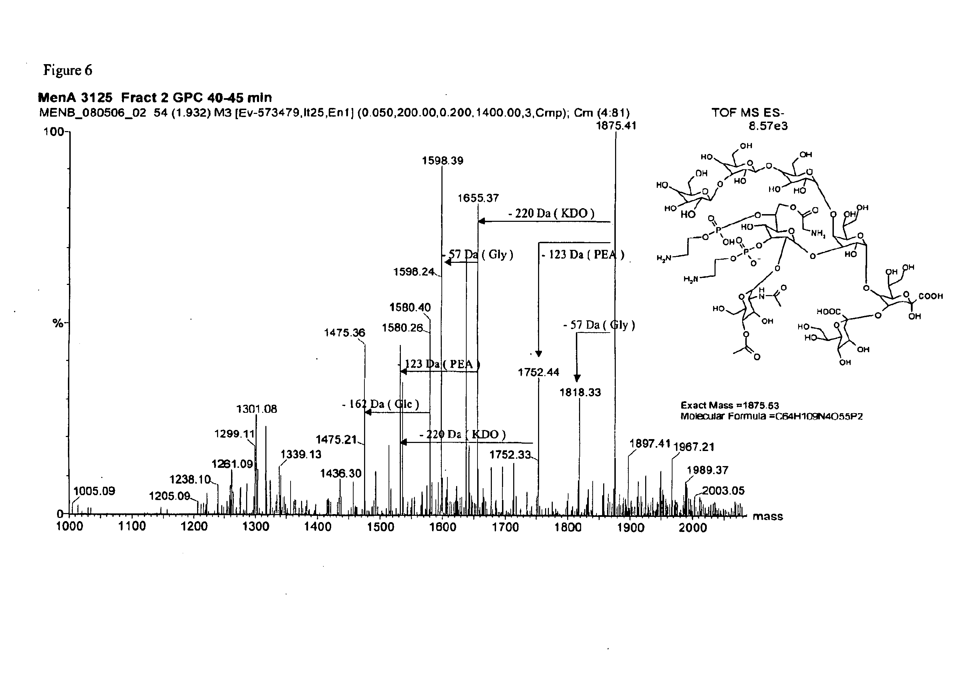

Figure 6: Mass spectrometry structural analysis of MenA 3125 L10 LOS.

### DESCRIPTION OF THE INVENTION

The subject matter of and information disclosed within the publications and patents or patent applications mentioned in this specification are incorporated by reference herein.

Reference to "lipooligosaccharide" (or "LOS") may also be referred to as "lipopolysaccharide" or "LPS".

The terms "comprising", "comprise" and "comprises" herein is intended by the inventors to be optionally substitutable with the terms "consisting of", "consist of", and "consists of", respectively, in every instance.

The present inventors have found that shortening the LOS oligosaccharide structures leads to the loss of epitopes that can elicit a bacteriocidal immune response. Instead, the inventors have found that in order to use LOS most effectively in a vaccine formulation, the LOS oligosaccharide structure must be retained as much as possible, but a combination of just 2 (or 3 or 4) LOS antigens can yield a universally effective Neisserial (preferably meningococcal) accine. A first aspect of the invention is a vaccine comprising L3V LOS and L3 LOS from Neisserial strains, wherein the L2 L3V and the L3 LOS has the following structure: 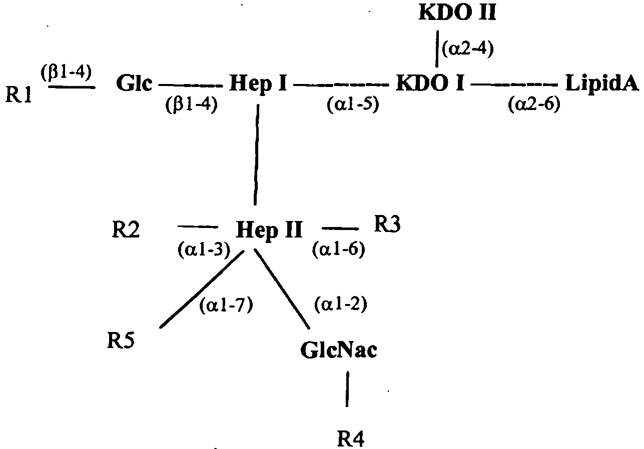 and wherein for the L3V LOS: 
GlcNac — Gal —    Gal — GlcNac — Gal —
NeuNac — Gal — GlcNac — Gal —
(α2-3) (β1-4) (β1-3)
(β1-3)    (β1-4)    (β1-3)
R1=    ,    ,or    ,
R2= PEA,
R3= PEA,
R4= H or OAc,
RS=H, PEA, or Gly,
and wherein for the L3 LOS: 
R1= GlcNac — Gal — , Gal — GlcNac — Gal — ,or NeuNac — Gal — GlcNac — Gal — ,
(β1-3) (β1-4) (β1-3) (α2-3) (β1-4) (β1-3)
R2= PEA,
R3= H,
R4= H or OAc,
R5=H or Gly
further comprising a pharmaceutically acceptable excipient.

Such vaccines are useful for the prevention or treatment of Neisserial (preferably meningococcal or meningococcal B) disease, and may further comprise L10 and/or L4 LOS of the invention. LOS may be isolated by either known purification procedures, or may be present in at least 2 outer membrane vesicle (or bleb) preparations derived from, for instance, L3V and L3 Neisserial strains. In order to remove toxic loosly held LOS from the bleb preparation, but retain high levels of integrated LOS antigen in the bleb, it is preferred that the blebs are extracted using a low concentration of detergent - 0-0.3%, preferably 0.05-0.2%, most preferably around 0.1%, preferably deoxycholate (or DOC). Such a combination of LOS antigens, particularly in a bleb vaccine, is surprisingly advantageous in being effective against over 90% of *N meningitidis* strains.

The inventors have also found that the above bleb vaccines of the invention, and indeed any Neisserial (preferably gonococcal or meningococcal) derived bleb immunogenic composition, can have an enhanced effect of protective antigens (including LOS) on their surface if certain combinations of immunodominant outer membrane proteins are downregulated in expression (and preferably deleted). A further aspect of the invention is therefore one or more Neisserial bleb preparation of the invention being derived from a neisserial strain which has had 2 or more of the following outer membrane proteins downregulated in expression, and preferably deleted, compared to the native, non-modified strain: PorA, PorB, OpA, OpC or PilC. Preferably PorA and OpA, PorA and OpC, OpA and OpC, or PorA & OpA & and OpC are downregulated or deleted. Downregulation (preferably deletion) of expression of FrpB has also been shown to be beneficial in enhancing the effect of cross-protective antigens - particulary in bleb preparations made from neisserial strains grown in iron limiting conditions. A Neisserial bleb of the invention derived from a strain with this mutation is thus a further embodiment of the invention, as are blebs derived from a combination of FrpB downregulation with one or more of the downregulations mentioned above. It is preferred that if PorA is downregulated PorB should not be downregulated, and vice versa.

The above mutations are beneficial in any Neisserial (preferably meningococcal, most preferably menB) strain from which bleb immunogenic compositions or vaccines of the invention are to be derived, particularly those described herein, however it is preferred that L2, L3V or L3 immunotype Neisserial (preferably meningococcal, most preferably menB) strains are used, typically extracted with a low DOC % extraction process as described herein. Preferably the bleb immunogenic compositions or vaccine of the invention contain both L2 or L3V and L3 blebs where at least one (and preferably both) is deficient in the above combinations of immunodominant outer membrane proteins (or OMPs). Techniques for downregulating these genes are discussed in WO 01/09350 (incorporated by reference herein). Four different Opa genes are known to exist in the meningococcal genome (Aho et al. 1991 Mol. Microbiol. 5:1429-37), therefore where Opa is said to be downregulated in expression it is meant that preferably 1, 2, 3 or (preferably) all 4 genes present in meningococcus are so downregulated. Such downregulation may be performed genetically as described in WO 01/09350 or by seeking readily-found, natural, stable meningococcal strains that have no or low expression from the Opa loci. Such strains can be found using the technique described in Poolman et al (1985 J. Med. Micro. 19:203-209) where cells that are Opa⁻ have a different phenotype to cells expressing Opa which can be seen looking at the appearance of the cells on plates or under a microscope. Once found, the strain can be shown to be stably Opa⁻ by performing a Western blot on cell contents after a fermentation run to establish the lack of Opa.

### Safety of the above LOS immunogenic compositions or vaccines

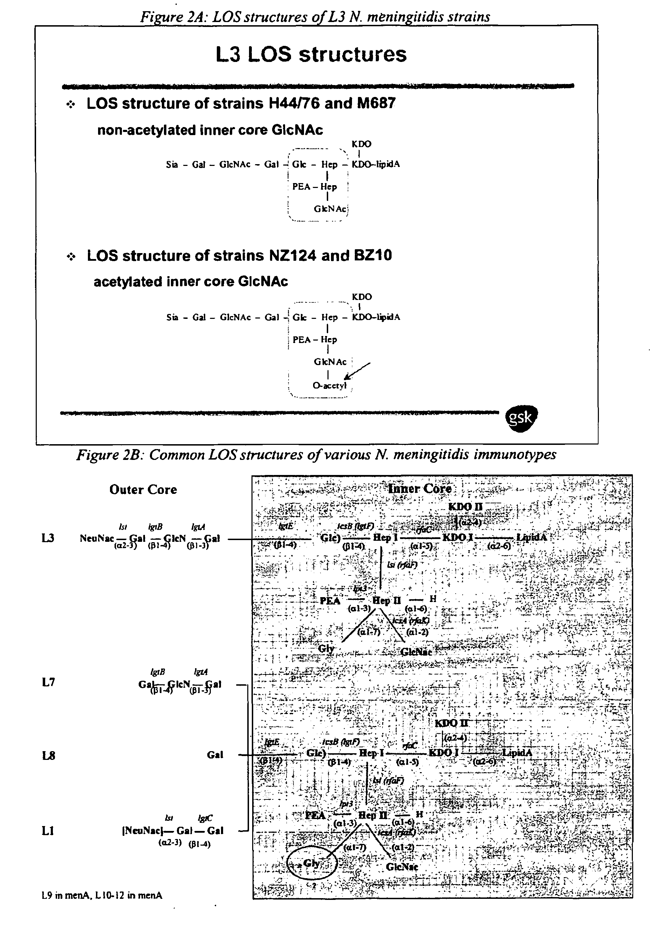

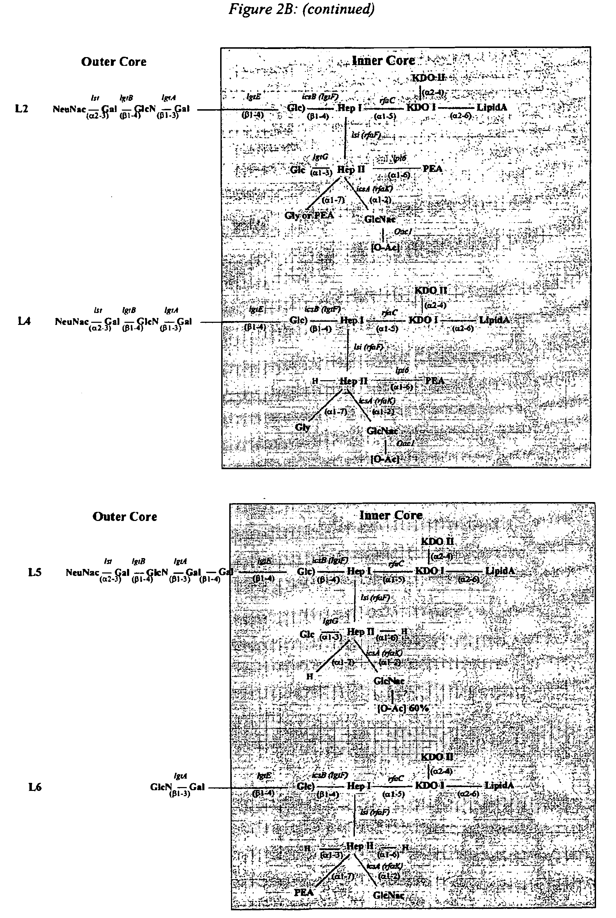

The safety of antibodies raised to L3 or L2 LOS has been questioned, due to the presence of a structure similar to the lacto-N-neotetraose oligosaccharide group (Galβ1-4GlcNAcβ1-3Galβ1-4Glcβ1- ; Fig 1) present in human glycosphingolipids. Although a large number of people have been safely vaccinated with deoxycholate extracted vesicle vaccines containing residual amount of L3 LOS (G. Bjune et al, Lancet (1991), 338, 1093-1096; GVG. Sierra et al, NIPH ann (1991), 14, 195-210), if LOS is to be retained as an antigen as discussed herein, the deletion of a terminal part of the LOS saccharide structure has been found by the current inventors to be advantageous in preventing cross-reaction of the anti-LOS immune response with structures present at the surface of human tissues. In a preferred embodiment, inactivation of the *lgtB* gene results in an intermediate LOS structure in which the terminal galactose residue and the sialic acid are absent (see figures 1 and 2, the mutation leaves a 4GlcNAcβ1-3Galβ1-4Glcβ1- structure in L2 and L3 and L4 LOS). Such intermediates could be obtained in an L3 and/or an L2 (and/or an L4) LOS strain. An alternative and less preferred (short) version of the LOS can be obtained by turning off the *lgtE* gene. A further alternative and less preferred version of the LOS can be obtained by turning off the lgtA gene. If such an lgtA⁻ mutation is selected it is preferred to also turn off lgtC expression to prevent the non-immunogenic L1 immunotype being formed.

LgtB⁻ mutants are most preferred as the inventors have found that this is the optimal truncation for resolving the safety issue whilst still retaining an LOS protective oligosaccharide epitope that can still induce a bactericidal (and even cross-bactericidal) antibody response.

Therefore, the above L2 or L3V and/or L3 preparations (or one or more of the LOS of the invention as defined above) (whether purified or in an isolated bleb) of the invention or meningococcal bleb preparations of the invention in general (particularly L2 or L3V and/or L3) are advantageously derived from a Neisserial strain (preferably meningococcal) that has been genetic engineered to permanently downregulate the expression of functional gene product from the lgtB, lgtA or lgtE gene, preferably by switching the gene off, most preferably by deleting all or part of the promoter and/or open-reading frame of the gene.

Preferably the neisserial strains of the invention are deficient in synthesising capsular polysaccharide.

Where the above bleb preparations of the invention are derived from a meningococcus B strain, it is particularly preferred that the capsular polysaccharide (which also contains human-like saccharide structures) is also removed. Although many genes could be switched off to achieve this, the inventors have advantageously shown that it is preferred that the bleb production strain has been genetically engineered to permanently downregulate the expression of functional gene product from the siaD gene (i.e. downregulating α-2-8 polysialyltransferase activity), preferably by switching the gene off, most preferably by deleting all or part of the promoter and/or open-reading frame of the gene. Such an inactivation is described in WO 01/09350. The siaD (also known as synD) mutation is the most advantageous of many mutations that can result in removing the human-similar epitope from the capsular polysaccharide, because it one of the only mutations that has no effect on the biosynthesis of the protective epitopes of LOS, thus being advantageous in a process which aims at ultimately using LOS as a protective antigen, and has a minimal effect on the growth of the bacterium. A preferred aspect of the invention is therefore a bleb immunogenic preparation or vaccine as described above which is derived from an lgtE siaD⁻, an lgtA⁻ siaD⁻ or, preferably, an lgtB⁻ siaD⁻ meningococcus B mutant strain. The strain itself is a further aspect of the disclosure.

Although siaD⁻ mutation is preferable for the above reasons, other mutations which switch off meningococcus B (or meningococcus in general) capsular polysaccharide synthesis may be used. Thus bleb production strain can be genetically engineered to permanently downregulate the expression of functional gene product from one or more of the following genes: ctrA, ctrB, ctrC, ctrD, synA (equivalent to synX and siaA), synB (equivalent to siaB) or synC (equivalent to siaC) genes, preferably by switching the gene off, most preferably by deleting all or part of the promoter and/or open-reading frame of the gene. The lgtE⁻ mutation may be combined with one or more of these mutations. Preferably the lgtB⁻ mutation is combined with one or more of these mutations. A further aspect of the invention is therefore a bleb immunogenic preparation or vaccine as described above which is derived from such a combined mutant strain of meningococcus B (or meningococcus in general). The strain itself is a further aspect of the disclosure.

A Neisserial locus containing various lgt genes, including lgtB and lgtE, and its sequence is known in the art (see M. P. Jennings et al, Microbiology 1999, 145, 3013-3021 and references cited therein; J. Exp. Med. 180:2181-2190 [1994]; WO 96/10086).

Where full-length (non-truncated) LOS of the invention is to be used in the final product, it is desirable for the LOS not to be sialyated (as such LOS may generate an immune response against the most dangerous, invasive meningococcal B strains which are also unsialylated). In such case using a capsule negative strain which has a deleted synA (equivalent to synX and siaA), synB (equivalent to siaB) or synC (equivalent to siaC) gene is advantageous, as such a mutation also renders menB LOS incapable of being sialylated. In one embodiment of the invention the lst gene (Gilbert et al., JBC 1996, 271:28271-6) is rendered functionally inactive (e.g. through deleting or disrupting or reducing expression from the gene), or a strain is selected for the invention where the gene is naturally disrupted (for the L3 family of immunotypes, such a defective strain is often termed an L7 immunotype). Lst is an alpha-2,3-sialyltransferase which adds the terminal sialic acid to the LOS alpha chain (but has no effect on sialic acid production). In one embodiment the strain of the invention is lst(-) and siaD(-).

The above mutations are beneficial in any Neisserial (preferably meningococcal, most preferably menB) strain from which bleb immunogenic compositions or vaccines are to be derived, particularly those described herein, however it is preferred that L2 or L3 immunotype Neisserial (preferably meningococcal, most preferably menB) strains are used, typically extracted with a low DOC % extraction process as described herein. Preferably the bleb immunogenic compositions or vaccines of the invention contains both L2 or L3V and L3 blebs where at least one (and preferably both) is derived from strains deficient in the expression of the above genes.

### The Toxicily of LOS

The above purified LOS or bleb immunogenic compositions or vaccines of the invention may also be rendered less toxic by downregulating expression of certain genes in the bacterial production strain from which they are derived. Although such detoxification may not be necessary for intranasal immunization with native OMV (J.J. Drabick et al, Vaccine (2000), 18, 160-172), for parenteral vaccination detoxification would present an advantage. Preferably the purified LOS or bleb immunogenic compositions or vaccines of the invention are detoxified by genetically engineering the Neisserial production strain by mutation/modification/inactivation of the genes involved in LipidA biosynthesis, particularly those genes involved in adding secondary acyl chains to lipidA, in particular by downregulating the expression of functional gene product from the msbB and/or htrB genes, and preferably by switching the gene off, most preferably by deleting all or part of the promoter and/or open-reading frame of the gene. Alternatively (or in addition) the purified LOS or bleb immunogenic compositions or vaccines can be derived from a Neisserial strain which has been genetically modified so that one or more of the following genes are upregulated (by introducing a stronger promoter or integrating an extra copy of the gene): pmrA, pmrB, pmrE and pmrF. Alternatively (or in addition) the purified LOS or bleb immunogenic compositions or vaccines may be detoxified by adding non-toxic peptide functional equivalents of polymyxin B [a molecule with high affinity for Lipid A] to the compositions (see below - i.e. one or more of the LOS of the invention is complexed with a lipid A-binding peptide suitable for reducing the toxicity of the LOS, such as SAEP2 or SAEPII).

See WO 01/09350 for more detail on the above detoxification methods, and for relevant promoter / gene sequences and upregulation and downregulation methods. The msbB and htrB genes of Neisseria are also called lpxL1 and lpxL2, respectively, (see WO 00/26384) and deletion mutations of these genes are characterised phenotypically by the msbB⁻ mutant LOS losing one secondary acyl chain compared to wild-type (and retaining 4 primary and 1 secondary acyl chain), and the htrB⁻ mutant LOS losing both secondary acyl chains. Such mutations are preferably combined with mutations to ensure that the neisserial production strain is capsular polysaccharide deficient (see above) to ensure the optimal presentation of detoxified LOS on the bleb, or to aid the purification of the detoxified subunit LOS.

See WO 93/14115, WO 95/03327, WO2006/108586, Velucchi et al (1997) J Endotoxin Res 4: 1-12, and EP 976402 for further details of non-toxic peptide functional equivalents of polymyxin B (lipid A-binding peptides suitable for reducing the toxicity of the LOS of the invention) that may be used in the compositions of this invention - particularly the use of the peptide SAEP 2 (of sequence KTKCKFLKKC where the 2 cysteines form a disulphide bridge), and SAEP II (a peptide dimer described in claims 1-10 of WO2006/108586). Reference to such lipid A-binding peptides herein may refer to any of the specific or general formulae of peptides described in the claims or examples of the above cited patent applications.

By "downregulating the expression of functional gene product" it is meant herein that additions, deletions or substitutions are made to the promoter or open reading frame of the gene in question such that the biosynthetic activity of the total gene product reduces (by 60, 70, 80, 90, 95 or most preferably 100%). Clearly frameshift mutations may be introduced, or weaker promoters substituted, however most preferably most or all of the open reading frame and/or promoter is deleted to ensure a permanent downregulation of the (active) gene product (as described in WO 01/09350).

The above mutations are beneficial in any Neisserial (preferably meningococcal, most preferably menB) strain from which bleb immunogenic compositions or vaccines are to be derived, particularly those described herein, however it is preferred that L2 or L3 immunotype Neisserial (preferably meningococcal, most preferably menB) strains are used, typically extracted with a low DOC % extraction process as described herein. Preferably the bleb immunogenic compositions or vaccines of the invention contain both L2 or L3V and L3 blebs (or the bleb preprations of the invention) where at least one (and preferably both) is derived from strains deficient in the expression of the above genes.

Further aspects of the invention include the above described genetically modified Neisserial (preferably meningococcal or gonococcal or meningococcal B) strains from which the LOS or bleb immunogenic preparations or vaccines of the invention may be derived.

### The LOS or LOS-containing bleb preparations of the invention

A further aspect of the invention is a LOS preparation (particularly any of those described above) isolated from the Neisserial strains of the invention. Preferably the isolated LOS (or LOS-containing bleb) is L2 or L3 immunotype, and preferably the immunogenic compositions or vaccines of the invention comprise both L2 or L3V and L3 LOS (or bleb) preparations of the invention.

Such preparations may also be improved by conjugating the oligosaccharide portion of the above LOS (whether purified or present in a bleb preparation) to a carrier comprising a source of T-cell epitopes (thus rendering the LOS an even better [T-dependent] immunogen). A purified LOS preparation of the invention may alternatively (or in addition) be rendered a better antigen by presenting it in liposome formulations known in the art (see for instance WO 96/40063 and references cited therein).

The process of isolation of LOS from bacteria is well known in the art (see for instance the hot water-phenol procedure of Wesphal & Jann [Meth. Carbo. Chem. 1965, 5:83-91]). See also Galanos et al. 1969, Eur J Biochem 9:245-249, and Wu et al. 1987, Anal Bio Chem 160:281-289. Techniques for conjugating isolated LOS are also known (see for instance EP 941738 incorporated by reference herein).

For the purposes of this invention "a carrier comprising a source of T-cell epitopes" is usually a peptide or, preferably, a polypeptide or protein. Conjugation techniques are well known in the art. Typical carriers include protein D from non typeable *H. influenzae,* tetanus toxoid, diphtheria toxoid, CRM197, or outer membrane proteins present in bleb (particularly neisserial or meningococcal) preparations.

Preferred isolated LOS compositions of the invention are: a composition comprising L2 or L3V and L3 isolated LOS wherein the oligosaccharide portion of each LOS is optionally conjugated to a carrier comprising a source of T-cell epitopes, a composition comprising L2, L3V or L3 LOS which has a structure consistent with it having been derived from a lgtB⁻ meningococcal strain wherein the oligosaccharide portion of each LOS is optionally conjugated to a carrier comprising a source of T-cell epitopes, and most preferably a composition comprising L2 or L3V and L3 isolated LOS which have a structure consistent with them having been derived from an IgtB⁻ meningococcal strain, wherein the oligosaccharide portion of each LOS is optionally conjugated to a carrier comprising a source of T-cell epitopes.

Preferably the LOS compositions of the invention (or or more or the LOS of the invention) have been detoxified. This may be done by known techniques of hydrazine or alkaline hydrolysis chemical treatments which remove acyl chains from the molecule (but which may reduce the protective efficacy of the molecule), but is preferably done by isolating the LOS from an htrB⁻ or msbB⁻ meningococcal mutant (as described above; particularly in capsule polysaccharide minus strains), or by adding a non-toxic peptide functional equivalent of polymyxin B [a molecule with high affinity to Lipid A] to the composition, in particular SAEP 2 or SAEPII (as described above).

The LOS of the invention may be administered in an isolated state (usually in the form of micelles if the lipid A moiety is still intact), or may be administered in a liposome. In such case outer membrane proteins may be added to the liposome, and the LOS may be conjugated intra-liposome to such outer membrane proteins to render the oligosaccharide a T-dependent antigen. This may be done with a similar chemistry as described for intra-bleb LOS cross-linking as described below.

### Intra-bleb cross-linking (conjugation) of the oligosaccharide portion of LOS to outer membrane proteins present on the surface of the bleb

Where LOS (in particular the LOS of the invention) is present in a bleb formulation the LOS is preferably conjugated in situ by methods allowing the conjugation of LOS to one or more outer membrane proteins also present on the bleb preparation (e.g. PorA or PorB in meningococcus). Thus a further aspect of the invention is a bleb preparation (one or more bleb preparations of the invention) from a Gram-negative bacterial strain in the outer-membrane of which is integrated an outer-membrane protein conjugated to LOS. Although LOS may be added to a bleb preparation for conjugation, it is preferred that the LOS is naturally present on the surface of the bleb preparation.

This process can advantageously enhance the stability and/or immunogenicity (providing T-cell help) and/or antigenicity of the LOS antigen within the bleb formulation - thus giving T-cell help for the T-independent oligosaccharide immunogen in its most protective conformation - as LOS in its natural environment on the surface of the outer membrane. In addition, conjugation of the LOS within the bleb can result in a detoxification of the LOS (without wishing to be bound by theory, the Lipid A portion may be more stably buried in the outer membrane if conjugated thus being less available to cause toxicity). Thus the detoxification methods mentioned above of isolating blebs from htrB⁻ or msbB⁻ mutants, or by adding non toxic peptide functional equivalent of polymyxin B to the composition may not be required (but which may be added in combination for additional security).

The conjugated bleb preparations of the invention are typically such that the toxicity of the LOS in the bleb is reduced compared to the same blebs with the same amount of totally unconjugated LOS. LOS toxicity may be readily determined by a skilled person, for example using the LOS rabbit pyrogenicity assay in the European Pharmacopoeia (see Example 7 of W02004/014417).

The conjugated bleb preparations of the invention are advantageously such that the conjugated LOS has a conformation suitable for eliciting an immune response in a host, the sera from which is reactive (can bind) with unconjugated LOS - preferably present on the bacterium from which the bleb preparation was made, and most preferably in a bactericidal fashion in a SBA assay.

Where neisserial blebs are conjugated to LOS, and the blebs are derived from a strain downregulated in one or more immunodominant outer membrane proteins as described herein, it is preferred that if PorA is downregulated PorB should not be downregulated, and vice versa. This allows the majority of LOS to cross-link with a major outer membrane protein, and thus minimises any effect of conjugation on cross-protective minor outer membrane antigens present in the bleb.

In particular, the inventors have found that a composition comprising blebs wherein LOS present in the blebs has been conjugated in an intra-bleb fashion to outer membrane proteins also present in the bleb can form the basis of a vaccine for the treatment or prevention of diseases caused by the organism from which the blebs have been derived, wherein such vaccine is of reduced toxicity (preferably substantially non-toxic) and/or is capable of inducing a T-dependent bactericidal response against LOS in its native environment.

This invention therefore further provides such an intra-bleb LOS conjugated bleb preparation. By "intra bleb" it is meant that LOS naturally present in the bleb is conjugated to outer membrane protein present on the same bleb.

Such bleb preparations may be isolated from the bacteria in question (see WO 01/09350), and then subjected to known conjugation chemistries to link groups (e.g. NH₂ or COOH) on the oligosaccharide portion of LOS to groups (e.g. NH₂ or COOH) on bleb outer membrane proteins. Cross-linking techniques using glutaraldehyde, formaldehyde, or glutaraldehyde/formaldehyde mixes may be used, but it is preferred that more selective chemistries are used such as EDAC or EDAC/NHS (J.V. Staros, R.W. Wright and D. M. Swingle. Enhancement by N-hydroxysuccinimide of water-soluble carbodiimide-mediated coupling reactions. Analytical chemistry 156: 220-222 (1986); and Bioconjugates Techniques. Greg T. Hermanson (1996) pp173-176). Other conjugation chemistries or treatments capable of creating covalent links between LOS and protein molecules that could be used in this invention are described in EP 941738.

Preferably the bleb preparations are conjugated in the absence of capsular polysaccharide. The blebs may be isolated from a strain which does not produce capsular polysaccharide (naturally or via mutation), or may be purified from most (more than 60, 70, 80, 90, or 99% removed) and preferably all contaminating capsular polysaccharide. In this way, the intra-bleb LOS conjugation reaction is much more efficient.

Preferably more than 5, 10, 20, 30, 40, 50, 60, 70, 80, 90, or 95% of the LOS present in the blebs is cross-linked/conjugated.

Preferably the blebs of the invention have been prepared such that the LOS content of the blebs is 3-30, 5-25, 10-25, 15-22, and most preferably around or exactly 20% LOS content as measured by silver staining after SDS-PAGE electrophoresis using purified LOS as a standard (see method of Tsai, J. Biol. Standardization (1986) 14:25-33). 20% LOS in meningococcal blebs can be achieved with a 0.1% low DOC extraction, which may remove losely held LOS molecules, but conserve the majority of the antigen.

Where the intra-bleb conjugated blebs are derived from meningococcus, it is preferred that the strain from which they are derived is a mutant strain that cannot produce capsular polysaccharide (e.g. one of the mutant strains described above, in particular siaD⁻ ). It is also preferred that immunogenic compositions or vaccines effective against meningococcal disease comprise both an L2 or L3V and L3 bleb, wherein the L2 or L3V and L3 LOS are both conjugated to bleb outer membrane proteins. Furthermore, it is preferred that the LOS structure within the intra-bleb conjugated bleb is consistent with it having been derived from an lgtB⁻ meningococcal strain. Most preferably immunogenic compositions or vaccines comprise intrableb-conjugated blebs: derived from a L2, L3V or L3 mutant meningococcal strain that cannot produce capsular polysaccharide and is lgtB⁻; comprising L2 or L3V and L3 blebs derived from mutant meningococcal strains that cannot produce capsular polysaccharide; comprising L2 or L3V and L3 blebs derived from mutant meningococcal strains that are lgtB⁻; or most preferably comprising L2 or L3V and L3 blebs derived from mutant meningococcal strains that cannot produce capsular polysaccharide and are lgtB⁻.

A typical L3 meningococcal strain that can be used for the present invention is the H44/76 menB strain. A typical L2 strain is the B16B6 menB strain or the 39E meningococcus type C strain or strain 760676. A typical L10 strain is the 3125 menA strain. An L4 strain is the C19 MenC strain.

As stated above, the blebs of the invention have been detoxified to a degree by the act of conjugation, and need not be detoxified any further, however further detoxification methods may be used for additional security, for instance by using blebs derived from a meningococcal strain that is htrB⁻ or msbB⁻ or adding a non-toxic peptide functional equivalent of polymyxin B [a molecule with high affinity to Lipid A] (preferably SEAP 2 or SAEP II) to the bleb composition (as described above). Conjugation of LOS (particularly in an intra-bleb fashion) thus surprisingly exhibits a lower toxicity of LOS compared with preparations comprising the same amount of unconjugated LOS. Thus a general method for detoxifying blebs (particularly meningococcal) is further provided by means of intra-bleb conjugation of LOS to bleb outer membrane protein, and a method for detoxifying LOS is also provided by means of conjugating the LOS to bleb outer membrane protein.

In the above way meningococcal blebs and immunogenic compositions or vaccines comprising blebs are provided which have as an important antigen LOS which is reduced in toxicity (and preferably substantially non-toxic), devoid of autoimmunity problems, has a T-dependent character, is present in its natural environment, and is capable of inducing a bactericidal antibody response against potentially more than 90% of meningococcal strains (in the case of L2 or L3V+L3 compositions).

One or more of Men A, C, Y or W capsular polysaccharides or oligosaccharides (preferably at least MenC, or MenA and MenC, or Men C and MenY) may also be conjugated onto an outermembrane protein of the bleb of the invention. Although this could be done in the same reaction as LOS cross-linking, it is preferred that this is done in a separate (preferably later) reaction.

The process of optimal intra-bleb LOS conjugation is a further aspect of the present invention. Said process should incorporate the steps of isolating blebs from a Gram negative bacterium (preferably using a low % of DOC as described herein), carrying out chemistry suitable for conjugating LOS (preferably via its oligosaccharide moiety) present in the blebs to an outer membrane protein present on the same bleb, isolating the intra-bleb conjugated bleb preparation, and optionally formulating the intra-bleb conjugated bleb preparation with a further intra-bleb conjugated bleb preparation made by the same process but having a different LOS immunotype (preferably mixing L2 and L3 Neisserial/meningococcal blebs) and/or formulating the bleb preparation with a pharmaceutically acceptable excipient to make a vaccine composition.

Intrableb conjugation should preferably incorporate 1, 2 or all 3 of the following process steps: conjugation pH should be greater than pH 7.0, preferably greater than or equal to pH 7.5 (most preferably under pH 9); conditions of 1-5% preferably 2-4% most preferably around 3% sucrose should be maintained during the reaction; NaCl should be minimised in the conjugation reaction, preferably under 0.1M, 0.05M, 0.01M, 0.005M, 0.001M, and most preferably not present at all. All these process features make sure that the blebs remain stable and in solution throughout the conjugation process.

The EDAC/NHS conjugation process is a preferred process for intra-bleb conjugation. EDAC/NHS is preferred to formalydehyde which can cross-link to too high an extent thus adversely affecting filterability. EDAC reacts with carboxylic acids (such as KDO in LOS) to create an active-ester intermediate. In the presence of an amine nucleophile (such as lysines in outer membrane proteins such as PorB), an amide bond is formed with release of an isourea by-product. However, the efficiency of an EDAC-mediated reaction may be increased through the formation of a Sulfo-NHS ester intermediate. The Sulfo-NHS ester survives in aqueous solution longer than the active ester formed from the reaction of EDAC alone with a carboxylate. Thus, higher yields of amide bond formation may be realized using this two-stage process. EDAC/NHS conjugation is discussed in J.V. Staros, R.W. Wright and D. M. Swingle. Enhancement by N-hydroxysuccinimide of water-soluble carbodiimide-mediated coupling reactions. Analytical chemistry 156: 220-222 (1986); and Bioconjugates Techniques. Greg T. Hermanson (1996) pp173-176. Preferably 0.01-5 mg EDAC / mg bleb is used in the reaction, more preferably 0.05-1 mg EDAC/mg bleb. The amount of EDAC used depends on the amont of LOS present in the sample which in turn depends on the deoxycholate (DOC) % used to extract the blebs. At low % DOC (e.g. 0.1%), high amounts of EDAC are used (1mg/mg and beyond), however at higher % DOC (e.g. 0.5%), lower amounts of EDAC are used (0.025-0. 1mg/mg) to avoid too much inter-bleb crosslinking.

A preferred process of the invention is therefore a process for producing intra-bleb conjugated LOS (preferably meningococcal) comprising the steps of conjugating blebs in the presence of EDAC/NHS at a pH between pH 7.0 and pH 9.0 (preferably around pH 7.5), in 1-5% (preferably around 3%) sucrose, and optionally in conditions substantially devoid of NaCl (as described above), and isolating the conjugated blebs from the reaction mix.

The reaction may be followed on Western separation gels of the reaction mixture using anti-LOS (e.g. anti-L2 or anti-L3) mAbs to show the increase of LOS molecular weight for a greater proportion of the LOS in the blebs as reaction time goes on.

Yields of 99% blebs can be recovered using such techniques.

EDAC was found to be an excellent intra-bleb cross-linking agent in that it cross-linked LOS to OMP sufficiently for improved LOS T-dependent immunogenicity, but did not cross link it to such a high degree that problems such as poor filterability, aggregation and inter-bleb cross-linking occurred. The morphology of the blebs generated is similar to that of unconjugated blebs (by electron microscope). In addition, the above protocol avoided an overly high cross-linking to take place (which can decrease the immunogenicity of protective OMPs naturally present on the surface of the bleb e.g. TbpA or Hsf).

### Techniques for isolating blebs

Outer Membrane Vesicles (OMVs or blebs) of the invention can be isolated by many known techniques (Fredriksen et al, NIPH Annals (1991), 14, 67-79; Zollinger et al, J. Clin Invest (1979), 63, 836-848; Saunders et al, Infect Immun (1999), 67, 113-119; J.J. Drabick et al, Vaccine (1999), 18, 160-172). These divide into 2 main groups - techniques which use deoxycholate (about 0.5%) to extract blebs from meningococcus, and techniques that use low levels of deoxycholate (DOC) or no deoxycholate at all. DOC free process blebs have the interesting feature of maintaining high level of LOS in the OMV - which is advantageous in a vaccine where LOS is a protective antigen. Compared to DOC extracted blebs, the concentration of L3 Ags in OMV obtained by a DOC free process is approximately ten times higher. A detergent-free (preferably DOC-free) process of preparing blebs is preferred for the purposes of the processes of this invention for this reason, although extraction with a buffer containing low levels of detergent (preferably DOC) may also be advantageous in that the step would leave most of the tightly interacting LOS in the bleb whilst removing any more toxic loosely retained LOS. Typically 0-0.5% and preferably 0.02-0.4%, 0.04-3% or 0.06-2% detergent (preferably DOC) is used for bleb extraction, more preferably 0.08-0.15%, and most preferably around or exactly 0.1% is used to obtain an optimal amount of LOS to be stably present in the blebs. DOC free (or low DOC - 0.3% DOC or under) extraction processes are particularly preferred where the LOS has been detoxified by one or more of the methods detailed above.

It is preferred that the LOS content of the blebs in all embodiments of the present invention is 3-30, 5-25, 10-25, 15-22, and most preferably around or exactly 20% LOS content as measured by silver staining after SDS-PAGE electrophoresis using purified LOS as a standard (see method of Tsai, J. Biol. Standardization (1986) 14:25-33). Using Nmen L3 LOS as a standard in this method, in general LOS content in Nmen L3 immunotype blebs extracted with 0.1% DOC is about 20% LOS, with 0.2% DOC is about 15% LOS, with 0.3% DOC is about 10% LOS, and with 0.5% DOC is about 5% LOS.

Bleb production can be carried out using any appropriate technique for separating blebs from cells or cell debris (e.g. through low speed centrifugation). Bleb preparations can be further purified through the use of ultracentrifugation (pelleting the blebs), or with the gentler techniques of ultrafiltration and/or diafiltration as described by Frasch et al. "Outer membrane protein vesicle vaccines for meningococcal disease" in Methods in Molecular Medicine, vol 66, Meningococcal Vaccines: Methods and Protocols 2001 pp81-107 (Edited by A.J. Pollard and M.C. Maiden, Humana Press Totowa, NJ).

### Vaccine Compositions

The immunogenic compositions of the invention may readily be formulated as vaccine compositions by adding a pharmaceutically acceptable excipient.

A process for making the Neisserial (preferably meningococcal) immunogenic compositions or vaccines of the invention is further provided comprising the steps of isolating, purified LOS of the invention as described above or producing isolated blebs of the invention as described above, and formulating the LOS or blebs with a pharmaceutically acceptable excipient. Preferably purified LOS of both immunotype L2 and L3 of the invention, or blebs of both immunotype L2 and L3 of the invention, or a purified LOS of L2 or L3V and a bleb of L3 (or vice versa), are combined in a mixing step. Preferably the purified LOS or bleb of the invention has been conjugated as decribed above after isolation. An additional liposome formulation step may also be added for the purified LOS (using techniques known in the art - see for instance WO 96/40063 and references cited therein). Preferably bleb preparations are isolated by extraction with low (or no) concentrations of DOC (as described above).

Such L2 or L3V and L3 combination processes can yield a vaccine which is effective against almost all meningococcal B strains.

The above immunogenic compositions or vaccines (or processes) may have added one or more (2, 3 or 4) meningococcal polysaccharides or oligosaccharides (either plain or conjugated to a carrier comprising T-cell epitopes, as described above) from serogroups A, C, Y or W to the composition. Preferably at least C is added (most preferably conjugated), and more preferably A and C or Y and C (preferably all conjugated) and most preferably A, C, Y and W (preferably all conjugated). Advantageously a conjugated *N. influenzae* B capsular polysaccharide or oligosaccharide is also included in the above compositions to generate a universal meningitis vaccine.

Preferably compositions consisting of or comprising compositions specifically individualised in WO 94/08021 are not claimed in the present invention. Optionally, compositions consisting of or comprising compositions specifically individualised in US2006/0047106 are not claimed in the present invention.

### Vaccine Formulations of the invention

The immunogenic compositions of the invention may be formulated with a suitable adjuvant to generate vaccine compositions of the invention.

Suitable adjuvants include an aluminium salt such as aluminum hydroxide gel (alum) or aluminium phosphate (preferably aluminium hydroxide), but may also be a salt of calcium (particularly calcium carbonate), iron or zinc, or may be an insoluble suspension of acylated tyrosine, or acylated sugars, cationically or anionically derivatised polysaccharides, or polyphosphazenes.

Suitable Th1 adjuvant systems that may be added include, Monophosphoryl lipid A, particularly 3-de-O-acylated monophosphoryl lipid A (or other non-toxic derivatives of LPS), and a combination of monophosphoryl lipid A, preferably 3-de-O-acylated monophosphoryl lipid A (3D-MPL) [or non toxic LPS derivatives] together with an aluminium salt (preferably aluminium phosphate). An enhanced system involves the combination of a monophosphoryl lipid A and a saponin derivative particularly the combination of QS21 [or other saponin] and 3D-MPL [or non toxic LPS derivative] as disclosed in WO 94/00153, or a less reactogenic composition where the QS21 [or saponin] is quenched with cholesterol as disclosed in WO96/33739. A particularly potent adjuvant formulation involving QS21, 3D-MPL and tocopherol in an oil in water emulsion is described in WO95/1721 and is a preferred formulation that may be added. Other adjuvants that may be added comprise a saponin, more preferably QS21 and/or an oil in water emulsion and tocopherol. Unmethylated CpG containing oligo nucleotides (WO 96/02555) may also be added.

Vaccine preparation is generally described in Vaccine Design ("The subunit and adjuvant approach" (eds Powell M.F. & Newman M.J.) (1995) Plenum Press New York).

An immunoprotective dose of vaccines can be administered via the systemic or mucosal route. These administrations may include injection via the intramuscular, intraperitoneal, intradermal or subcutaneous routes; or via mucosal administration to the oral/alimentary (preferably intra-nasal administration), respiratory, genitourinary tracts. Typically bleb quantity in each vaccine dose is selected as an amount which induces an immunoprotective response without significant, adverse side effects in typical vaccinees. Such amount will vary depending upon which specific immunogen is employed and how it is presented. Generally, it is expected that each dose will comprise 1-100µg of each bleb or LOS of the invention, preferably 5-50µg, and most typically in the range 5 - 25µg.

### Further improvements to the bleb immunogenic compositions or vaccines of the invention

The above bleb compositions of the invention may be further improved in efficacy in vaccines of the invention if the Neisserial strain from which they are derived (including gonococcus, and preferably meningococcus, most preferably *N. meningitidis* B) have one or more of the following genes (encoding protective antigens) upregulated by inserting further copies of the gene into the genome, or introducing a stronger promoter upstream of the existing gene, or any of the other ways discussed in WO 01/09350 which are capable of inducing modified strains to make over 1.2, 1.5, 2, 3, 5 or 10 times the level of antigen as compared to the unmodified strain: NspA (WO 96/29412), Hsf or truncates thereof (WO 99/31132 & WO 01/55182; also known as NhhA), Hap (PCT/EP99/02766), OMP85 (WO 00/23595), PilQ (PCT/EP99/03603), PldA (PCT/EP99/06718), FrpB (WO 96/31618), TbpA (WO92/03467, US5912336, WO93/06861 and EP586266), TbpB (WO93/06861 and EP586266), NadA (Comanducci et al J. Exp. Med. 2002 195; 1445-1454; NMB 1994), FrpA/FrpC or portions in common between these antigens involving 5 or more repeat sequences (WO 92/01460; Thompson et al., (1993) J. Bacteriol. 175:811-818; Thompson et al., (1993) Infect. Immun.. 61:2906-2911), LbpA, LbpB

(PCT/EP98/05117), FhaB (WO98/02547 SEQ ID NO 38 [nucleotides 3083-9025]), HasR (PCT/EP99/05989), lipo02 (PCT/EP99/08315), Tbp2 (WO 99/57280; NMB 0460), MltA (WO 99/57280; NMB 0033), TspA (WO 00/03003), TspB (WO 00/03003), ctrA (PCT/EP00/00135), MafA (NMB 0652), MafB (NMB0643), Omp26 (NMB 0181), Adhesin X (NMB 0315), Adhesin Y (NMB 0995), Adhesin Z (NMB 1119), and OstA (NMB 0280). Examples of NMB sequences can be found in the database at www.neisseria.org. Where Hsf is mentioned herein, the term may be substitutable in every instance for Hsf truncates - in particular those disclosed in WO 01/55182.

It is particularly preferred if both Hsf and TbpA (Low or High, or both Low and High molecular weight forms [EP 586266]), or Hsf and OMP85, or OMP85 and TbpA (Low or High, or both Low and High molecular weight forms), or NspA and Hsf, or NspA and OMP85, or NspA and TbpA (Low or High, or both Low and High molecular weight forms) are both upregulated. Where 2 blebs are comprised in the composition, it is preferred that each bleb has different upregulations. If TbpA High and Low are both to be upregulated, it is preferable that these are upregulated in 2 separate blebs present in the composition derived from 2 strains that naturally comprise the 2 forms of TbpA. Most preferably, the 2 strains have L2 and L3 LOS immunotypes. TbpA may be upregulated genetically or by growing the neisserial/meningococcal production strains in iron limited conditions for instance in the presence of 50-70 µM Desferal (deferoxamine mesylate, available from Sigma). If the latter approach is taken, it is preferred that the FrpB gene expression is downregulated (preferably deleted) as this variable antigen may become immunodominant in blebs isolated from meningococcal strains isolated in Iron limited conditions.

In a preferred embodiment, the composition of the invention comprises an L3 bleb from a lgtB⁻ (or lst⁻) capsular polysaccharide⁻msbB⁻ strain preferably upregulated in TbpA High and Hsf and an L2 or L3V bleb from a lgtB⁻ (or lst⁻) capsular polysaccharide⁻msbB⁻ strain preferably upregulated in TbpA Low and Omp85. More preferably both blebs are additionally downregulated in PorA and/or FrpB expression, and optionally OpC and/or OpA expression. The blebs are most preferably isolated via a low DOC process as described above, and the LOS in both blebs is intra-bleb cross-linked to outer membrane protein.

### Ghost or Killed Whole cell vaccines

The inventors envisage that the above compositions and vaccines concerning blebs can be easily extended to processes concerning ghost or killed whole cell preparations and vaccines (with identical advantages). Methods of making ghost preparations (empty cells with intact envelopes) from Gram-negative strains are well known in the art (see for example WO 92/01791). Methods of killing whole cells to make inactivated cell preparations for use in vaccines are also well known. Therefore the compositions and vaccines involving blebs described throughout this document are envisioned to be applicable to the same compositions or vaccines comprising equivalent ghost and killed whole cell preparations of the invention.

### Serum bactericidal assays on the compositions of the invention

The serum bactericidal assay is the preferred method to assess synergistic relationships between antigens when combined in an immunogenic composition of the invention.

Such a synergistic response may be characterised by the SBA elicited by the combination of antigens being at least 50%, two times, three times, preferably four times, five times, six times, seven times, eight times, nine times and most preferably ten times higher than the SBA elicited by each antigen separately. Preferably SBA is measured against a homologous strain from which the antigens are derived and preferably also against a panel of heterologous strains. (See below for a representative panel for instance BZ10 (B:2b:P1.2) belonging to the A-4 cluster; B16B6 (B:2a:P1.2) belonging to the ET-37 complex; and H44/76 (B:15:P1.7,16)). SBA is the most commonly agreed immunological marker to estimate the efficacy of a meningococcal vaccine (Perkins et al. J Infect Dis. 1998, 177:683-691). Satisfactory SBA can be acertained by any known method. SBA can be carried out using sera obtained from animal models, or from human subjects.

A preferred method of conducting SBA with human sera is the following. A blood sample is taken prior to the first vaccination, two months after the second vaccination and one month after the third vaccination (three vaccinations in one year being a typical human primary vaccination schedule administered at, for instance, 0, 2 and 4 months, or 0, 1 and 6 months). Such human primary vaccination schedules can be carried out on infants under 1 year old (for instance at the same time as Hib vaccinations are carried out) or 2-4 year olds or adolescents may also be vaccinated to test SBA with such a primary vaccination schedule. A further blood sample may be taken 6 to 12 months after primary vaccination and one month after a booster dose, if applicable.

SBA will be satisfactory for an antigen or bleb preparation with homologous bactericidal activity if one month after the third vaccine dose (of the primary vaccination schedule) (in 2-4 year olds or adolescents, but preferably in infants in the first year of life) the percentage of subjects with a four-fold increase in terms of SBA (antibody dilution) titre (compared with pre-vaccination titre) against the strain of meningococcus from which the antigens of the invention were derived is greater than 30%, preferably greater than 40%, more preferably greater than 50%, and most preferably greater than 60% of the subjects.

Of course an antigen or bleb preparation with heterologous bactericidal activity can also constitute bleb preparation with homologous bactericidal activity if it can also elicit satisfactory SBA against the meningococcal strain from which it is derived.

SBA will be satisfactory for an antigen or bleb preparation with heterologous bactericidal activity if one month after the third vaccine dose (of the primary vaccination schedule) (in 2-4 year olds or adolescents, but preferably in infants in the first year of life) the percentage of subjects with a four-fold increase in terms of SBA (antibody dilution) titre (compared with pre-vaccination titre) against three heterologous strains of meningococcus is greater than 20%, preferably greater than 30%, more preferably greater than 35%, and most preferably greater than 40% of the subjects. Such a test is a good indication of whether the antigen or bleb preparation with heterologous bactericidal activity can induce cross-bactericidal antibodies against various meningococcal strains. The three heterologous strains should preferably have different electrophoretic type (ET)-complex or multi locus sequence typing (MLST) pattern (see Maiden et al. PNAS USA 1998, 95:3140-5) to each other and preferably to the strain from which the antigen or bleb preparation with heterologous bactericidal activity is made or derived. A skilled person will readily be able to determine three strains with different ET-complex which reflect the genetic diversity observed amongst meningococci, particularly amongst meningococcus type B strains that are recognised as being the cause of significant disease burden and/or that represent recognised MenB hyper-virulent lineages (see Maiden et al. *supra*). For instance three strains that could be used are the following: BZ10 (B:2b:P1.2) belonging to the A-4 cluster; B16B6 (B:2a:P1.2) belonging to the ET-37 complex; and H44/76 (B:15:P1.7,16) belonging to the ET-5 complex, or any other strains belonging to the same ET/Cluster. Such strains may be used for testing an antigen or bleb preparation with heterologous bactericidal activity made or derived from, for instance, meningococcal strain CU385 (B:4:P1.15) which belongs to the ET-5 complex. Another sample strain that could be used is from the Lineage 3 epidemic clone (e.g. NZ124 [B:4:P1.7,4]). Another ET-37 strain is NGP165 (B:2a:P 1.2).

Processes for measuring SBA activity are known in the art. For instance a method that might be used is described in WO 99/09176 in Example 10C. In general terms, a culture of the strain to be tested is grown (preferably in conditions of iron depletion - by addition of an iron chelator such as EDDA to the growth medium) in the log phase of growth. This can be suspended in a medium with BSA (such as Hanks medium with 0.3% BSA) in order to obtain a working cell suspension adjusted to approximately 20000 CFU/ml. A series of reaction mixes can be made mixing a series of two-fold dilutions of sera to be tested (preferably heat-inactivated at 56°C for 30 min) [for example in a 50µl/well volume] and the 20000 CFU/ml meningococcal strain suspension to be tested [for example in a 25µl/well volume]. The reaction vials should be incubated (e.g. 37°C for 15 minutes) and shaken (e.g. at 210 rpm). The final reaction mixture [for example in a 100µl volume] additionally contains a complement source [such as 25 % final volume of pretested baby rabbit serum, or human serum for human serology], and is incubated as above [e.g. 37°C for 60 min]. A sterile polystyrene U-bottom 96-well microtiter plate can be used for this assay. A aliquot [e.g. 10 µl] can be taken from each well using a multichannel pipette, and dropped onto Mueller-Hinton agar plates (preferably containing 1 % Isovitalex and 1 % heat-inactivated Horse Serum) and incubated (for example for 18 hours at 37°C in 5 % CO₂). Preferably, individual colonies can be counted up to 80 CFU per aliquot. The following three test samples can be used as controls: buffer + bacteria + complement; buffer + bacteria + inactivated complement; serum + bacteria + inactivated complement. SBA titers can be straightforwardly calculated using a program which processes the data to give a measurement of the dilution which corresponds to 50 % of cell killing by a regression calculation.

### EXAMPLES

The examples below are carried out using standard techniques, which are well known and routine to those of skill in the art, except where otherwise described in detail. The examples are illustrative, but do not limit the invention.

### Example 1:

Examples describing deletions genes encoding proteins involved in capsular polysaccharide production of meningococcus (e.g. MenB), the deletion of the PorA gene, the upregulation of various protective outer membrane proteins on the surface of meningococcal blebs, the downregulation of immunodominant proteins or biosynthetic enzymes (such as siaD(-) mutations), and processes for isolating blebs are described in WO 01/09350. Further information is given in WO 2004/014417 and WO 2004/014418. Note NMB and NMA gene sequence references herein refer to reference numbers to sequences which can be accessed from www.neisseria.org. A schematic showing the conventional structures of the LOS immunotypes is shown in Figure 1 (from Kahler et al. 2005 Glycobiology 15:409-419 / 2006 JBC 281:19939-19948). See also Figure 2B.

### Example 2: Inner-core LOS O-acetylation - potential impact on bactericidal titers

### Summary

- MS-MS analysis has shown that the N-acetyl-glucosamine (GlcNAc) of the inner-core LOS of strain NZ124 (L3) is O-acetylated. This is not the case for strains H44/76 and M687 as well as for the L3 lgtB(-) and L3 lst(-) vaccine strains (B1854= TrL3 and B1948= L7, respectively) derived from strain H44/76.
- Strain NZ124 is not more resistant to the antibody mediated complement killing than strains H44/76 and M687
- The accessibility of the low-exposed surface epitopes to bactericidal antibodies appears to be similar for strains NZ124 and H44/76.
- In five animal models using four animal species, and whatever the formulation used, the anti-TrL3 and anti-L7 blebs sera were less effective to mediate the killing of strain NZ124 compared to strains H44/76 and M687
- These results suggest that acetylation of the GlcNAc of inner-core LOS could reduce the efficacy of the killing mediated by anti-"non-acetylated LOS" antibodies.

### Introduction

Based on MS/MS analysis, two different structures of the L3 LOS of *Neisseria meningitidis* are described. These two structures are differentiated by the presence or not of an acetyl group on the GlcNAc of inner-core (Figure 2A). In the literature the L3 structure is described without this additional acetyl group.

The GlcNAc of the inner core LOS of TrL3 and L7 blebs is not acetylated. This is also the case for the wild type strains H44/76 and M97250687 (M687). In contrast, the WT strain NZ124 possesses an acetylated GlcNAc. These three WT serogroup B *N. meningitidis* strains are immunotyped as L3. NZ124 is a New Zealand epidemic strain isolated in 1998 and available from the New Zealand Institute of Environmental Science and Research, Wellington, New Zealand.

Sera from mice immunized with TrL3 or L7 blebs contain high levels of bactericidal antibodies against strains H44/76 and M687. However, these sera are less effective against strain NZ124. Indeed, the bactericidal titers measured with anti-L7/TrL3 blebs sera on strain NZ124 are 3-20 times lower than the titers measured on strains H44/76 and M687.

At least three hypotheses could explain the lower bactericidal antibody titers measured against strain NZ124:
- this strain could be intrinsically more resistant to the killing mediated by antibodies and complement than strains H44/76 and M687;
- on this strain, the LOS epitopes could be less accessible to the bactericidal antibodies;
- the acetylation of the inner core LOS could impact negatively on the efficacy of the killing mediated by anti-"non-acetylated" LOS antibodies.

### Results

### Is strain NZ124 more resistant to the killing mediated by antibodies?

In order to answer this question, we have analyzed in SBA the sera from mice immunized with different PorA+ blebs vaccines. The anti-sera have been tested in SBA against homologous and heterologous PorA strains. The results presented in Table 1 are from two experiments.

Immunization of mice with P1.7,16 blebs induced the production of bactericidal antibodies that were able to mediate the killing of homologous PorA P1.7,16 strain (titer of 1/2300 on H44/76) but not heterologous PorA strains (M687 and NZ124). A similar observation was made with the P1.19,15 vaccine which was only able to induce a protective bactericidal response against homologous PorA strain (titer of 1/900 on M687). Mice immunized with a vaccine containing the P1.7,4 PorA had high levels of bactericidal antibodies against strain NZ124 (titer of 1/6200) but not against strain H44/76.

**Table 1 : Impact of immunization with different PorA+ blebs on the induction of bactericidal antibodies against a panel of MenB strains expressing different PorA (GMTfor 50% killing).**

| | Vaccine strain(s) | | |
|---|---|---|---|
| Strains tested in SBA | H44/76 (P1.7,16) | CU385 (P1.19,15) | CU385-NZ228/98 (P1.19,15+P1.7,4) |
| H44/76 (P1.7, 16 | 2300 | <100 | <100 |
| M687 (P1.19,15) | <100 | 900 | 3800 |
| NZ124 (P1.7,4) | NT | <100 | 6200 |

In these experiments, the highest bactericidal antibody titers were measured against strain NZ124 suggesting that this strain is not more resistant to killing mediated by antibodies and complement than strains H44/76 and M687.

### Are the LOS epitopes of strain NZ124 well accessible to antibodies?

It has previously been suggested that the size of the capsule may limit the accessibility of antibodies to the NspA surface epitopes especially because the NspA loops are relatively small compared to, for example, the VR1 and VR2 loops of PorA. Indeed, a relation was established between the size of the capsule and the ability of anti-NspA sera to induce complement mediated killing (Moe et al, 1999 I&I 67:5664-75).

Because the LOS of *N. meningilidis* possesses a short saccharidic chain, the accessibility to its protective epitopes by antibodies could be impaired by the thickness of the capsule. To determine if such a mechanism could explain the lower bactericidal titers measured with anti-LOS antibodies on strain NZ124, bactericidal assays were performed with an anti-NspA MAb (MAb Me-7) on three different MenB strains including NZ124.

In presence of complement, strains H44/76 and NZ124 were easily killed by the MAb Me-7 as demonstrated by the bactericidal titers above 1/2560. In contrast, strain M687 was more resistant to the killing mediated by the anti-NspA MAb; a titer of only 1/347 was measured.

In conclusion, the accessibility of the protective NspA epitopes is similar for strains H44/76 and NZ124. Therefore, we can postulate that the lower efficacy of anti-TrL3 blebs antibodies in mediating the killing of strain NZ124 is not due to a lower accessibility of the protective LOS epitopes.

### Does the acetylation of the GlcNAc of inner-core LOS reduce the efficacy of anti-"non-acetylated" LOS bactericidal antibodies?

Mice, rabbits, guinea pigs, infant rats and adult rats were immunized with *por A* KO blebs. Most of the experiments were done with blebs obtained from strains producing penta-acylated lipidA LOS (*msbB* mutation, B1853, B1854 and B1948 blebs) but few experiments were also done with blebs containing hexa-acylated LOS (B1820 blebs). Different formulations were tested using aluminum salts (Al(OH)₃ or AlPO₄) or not (non-adsorbed formulations) and CpG. The anti-blebs sera were tested in SBA using baby rabbit complement against strains H44/76, M687 and NZ124.

The bactericidal titers measured with sera from animals immunized with TrL3 B 1820 blebs are shown in Table 2. Whatever the animal species the bactericidal titers obtained with strain NZ124 are lower than the bactericidal titers obtained with strains H44/76 and M687.

**Table 2 : Impact of the immunization of mice, guinea pigs and rabbits with B1820 blebs on the induction ofbactericidal antibodies against three MenB strains (GMT for 50% killing)**

| | Mice | | | Guinea pigs | | | Rabbits | | |
|---|---|---|---|---|---|---|---|---|---|
| Formulation | H44/76 | M687 | NZ124 | H44/76 | M687 | NZ124 | H44/76 | M687 | NZ124 |
| Al(OH)3 | 300 | 300 | 100 | 40 | 20 | <10 | 300 | 300 | 20 |
| AlPO4 | 3000 | 5000 | 800 | NT | NT | NT | NT | NT | NT |

Immunization with TrL3 *msbB* KO blebs (B1853 or B1854) or L7 *msbB* KO blebs (B1948) also induce higher serum bactericidal titers against strains H44/76 and M687 than against strain NZ124. This is observed in all the five different animal models tested: mice, infant rats and adult rats (Table 3a), guinea pigs and rabbits (Table 3b) and whatever the formulation used.

In conclusion, immunization of animals with blebs containing "non-acetylated LOS" elicited a lower bactericidal antibody response against "acetylated strain" (NZ124) than against "non-acetylated strains" (H44/76 and M687).

**Table 3a : Impact of the immunization of mice, infant rats and adult rats with penta-acetylated TrL3 (B1853 or B1854) and L7 (B1948) blebs on the induction of bactericidal antibodies against three MenB strains (GMT for 50% killing)**

| | | Mice | | | Infant rats | | | Rats | | |
|---|---|---|---|---|---|---|---|---|---|---|
| Formulation | Blebs | H44 | M687 | NZ124 | H44 | M687 | NZ124 | H44 | M687 | NZ124 |
| Non-ads | B1853/54 | 3000 | 1700 | 250 | 260 | 2000 | <20 | 450 | 5800 | <20 |
| | B1948 | 2600 | 2900 | 100 | 710 | 230 | 40 | 400 | 2500 | <20 |
| Al(OH)3 | B1853/54 | 2000 | 1200 | 280 | 30 | 60 | 10 | | | |
| | B1948 | 1600 | 940 | 140 | 320 | 480 | 10 | | | |
| CpG | B1853/54 | | | | 640 | 770 | 20 | | | |
| | B1948 | | | | 3300 | 1200 | 60 | | | |

**Table 3b : Impact of the immunization of guinea pigs and rabbits with penta-acetylated TrL3 (B1853 or B1854) and L7 (B1948) blebs on the induction of bactericidal antibodies against three MenB strains (GMT for 50% killing)**

| | | Guinea pigs | | | Rabbits | | |
|---|---|---|---|---|---|---|---|
| Formulation | Blebs | H44/76 | M687 | NZ124 | H44/76 | M687 | NZ124 |
| Non-ads | B1853/54 | 300 | 2200 | <20 | | | |
| | B1948 | 1000 | 500 | 30 | | | |
| Al(OH)3 | B1853/54 | | | | 400 | 400 | 30 |

### Discussions, conclusions and perspectives

Immunization of mice with TrL3 or L7 blebs elicited high level of bactericidal antibodies mediating the killing of strains H44/76 and M687. These antibodies were less bactericidal against strain NZ124 as the SBA titers measured with mouse sera were 3 to 20 times lower against strain NZ124 than against strain H44/76 and M687.

Preclinical data suggest that strain NZ124 is not more resistant to the antibody-complement-mediated killing than strains H44/76 and M687. In addition there is evidence suggesting that low surface exposed protective epitopes of NZ124 are not less accessible to antibodies than similar epitopes on the surface of strain H44/76. Electron microscopy and/or flow cytometry analyses could be done to confirm this finding.

One difference between the LOS of strain NZ124 and the LOS of strains H44/76 and M687 is the acetylation of the GlcNAc of inner-core which is only observed on NZ124 LOS. This difference could explain the lower efficiency of anti-TrL3 and anti-L7 blebs sera in mediating killing of strain NZ124 compared to strains H44/76 and M687. Indeed, it is known that the acetylation of protective saccharidic epitopes can positively or negatively influence the recognition of such epitopes by antibodies. However, the impact of acetylation/non-acetylation on the immunogenicity/antigenicity of protective epitopes described in one animal species is not always observed in other animal species and in humans.

The impact of the acetylation of saccharidic epitopes on their immunogenicity varies according to the "animal species" but also according to the antigens. Nevertheless, in the MenB case, similar observations have been made in the five animal models tested. These common observations across 4 different animal species suggest that the bactericidal antibodies induced by immunization with "non-acetylated LOS" could be less efficient in mediating the killing of "acetylated" strains such strain NZ124.

In order to confirm this hypothesis, a serum bactericidal assay using a second L3 acetylated strain will be developed (strain BZ10). In addition, the development of SBA using genetically modified strains such as an acetylated H44/76 strain and a de-acetylated NZ124 strain is also planned. Based on those results, new L3 blebs (with acetylated GlcNAc) could be evaluated.

### Example 3: Inner-core LOS O-acetylation - the neisserial gene for O-acetylation of LOS inner-core

On top of the sugar composition of the alpha-chain, "decoration" of heptose II seems to have an impact on LOS immunogenicity. PEA numbers and positions, presence of a Glucose in position 3, presence of a Glycine in position 7 and O-acetylation of GlcNac seem to be important determinants of cross-protection.

***lpt3*** gene (MacKinnon et al. 2002 Mol Microbiol. 43: 931-943) expresses the enzyme adding **PEA in position 3** on Heptose II. The gene (NMB2010) is not phase variable.

***lgtG*** gene expresses the enzyme adding a **Glucose in position 3** on Heptose II. This gene is phase variable (see WO04/015099) This gene is deleted in a number of *N. m.* strains, alone or in combination with *lpt6.*

***lpt6*** gene (Wright et al. 2004 J Bact. 186: 6970-6982) expresses the enzyme adding **PEA in position 6** on Heptose II. The gene (NMA0408) is deleted in a number of *N. m.* strains, alone or in combination with *lgtG.* The gene is not phase variable. *lpt6* and *lgtG* are located in the same region on the chromosome named lgt3.

Enzyme adding PEA in position 7 on Heptose II is unknown

Enzyme adding Glycine in position 7 on Heptose II is unknown

Enzyme adding O-Acetyl on GlcNAc was unknown until the present study.

### Identification of O-acetylation gene

- After BLAST studies with OafA protein (from *Salmonella;* homologous to *Haemophilus* O-acetylase Hi0391 + Hi0392) on translated Neisseria genomes (*N.gonorrhoeae, N.meningitidis* MenB MC58, MenC FAM18 and MenA Z2491), two families of genes were found in *Neisseria* named *oac1* (represented by the MC58 gene **NMB0285**) and *oac2* (represented by the MC58 gene **NMB1836**) with *oac1* family closer to OafA than *oac2.*
- Both genes are phase variable: presence of a polyG stretch in the ORF.
- In MC58 (non O-acetylated LOS), the *oac1* gene (NMB0285) is out of phase while the *oac2* (NMB 1836) is in phase.
- PCR products corresponding to *oac1* and *oac2* were obtained for each strain tested
- The polyG stretch sequenced to investigate functionality through polyG stretch.

Presence and functionality of NMB0285 and NMB1836 gene in SBA *N.m*. strains: Analytical data obtained by ARD with MS-MS (mass spectroscopy). Presence/functionality of the genes determined by molecular biology. Numbers in brackets are the numbers of G in the polyG stretch in the gene ORF.

| **strains** | **Stype** | **Itype** | **O-Ac (ARD)** | **oac1 (NMB0285)** | | **oac2 (NMB1836)** | |
|---|---|---|---|---|---|---|---|
| | | | | **presence (PCR)** | **functionality** | **presence (PCR)** | **functionality** |
| H44/76 | B | L3 | - | + | - (6) | + | + (10) |
| 760676 | B | L2 | + | + | + (5) | + | - (14) |
| NZ124 | B | L3 | + | + | + (5) | + | - (11) |
| BZ10 | B | L3 | + | + | + (5) | + | - (9) |
| M687 | B | L3 | - | + | - (6) | + | + (13) |
| B16B6 | B | L2 | + | + | +(5) | + | - (15) |
| 6275 (B2003) | B | L3V | + | + | + (5) | + | - (12) |
| 2986 | B | L2 | + | + | + (5) | + | + (10) |
| C11 (B1983) | C | L3V | + | + | + (5) | + | - (14) |
| C C19 | C | | + | + | + (5) | + | - (8) |
| YS1975 | Y | | + | + | + (5) | + | + (13) |
| Y MO1-0240539 | Y | | + | + | + (5) | + | - (12) |
| W 3193 | W | L3 | - | + | - (5*) | + | - |
| W S4383 | W | | + | + | + (5) | + | - (12) |
| A F8238 | A | L11 | + | + | + (5) | + | + (7) |
| A 3048 | A | | + | + | + (5) | + | + (10) |
| A3125 | A | L10 | + | + | + (5) | + | + (13) |

| | | | | | | | |
|---|---|---|---|---|---|---|---|
| * 1 substitution introducing a STOP before the G stretch | | | | | | | |

### Conclusion

- Perfect correlation (16/16) between the functionality of the NMB0285 gene and the detection of a O-acetyl group by MS-MS.
- No correlation (7/16) between the functionality of the NMB 1836 gene and the data obtained by MS-MS.
- We have strong evidence that the O-Acetylation gene in *Neisseria* is **NMB0285**

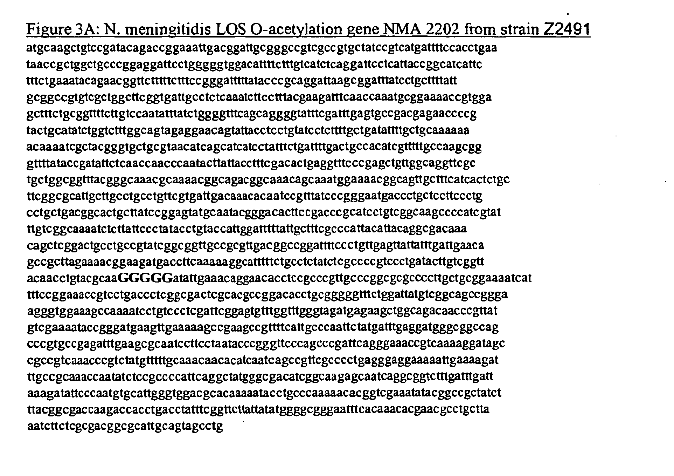

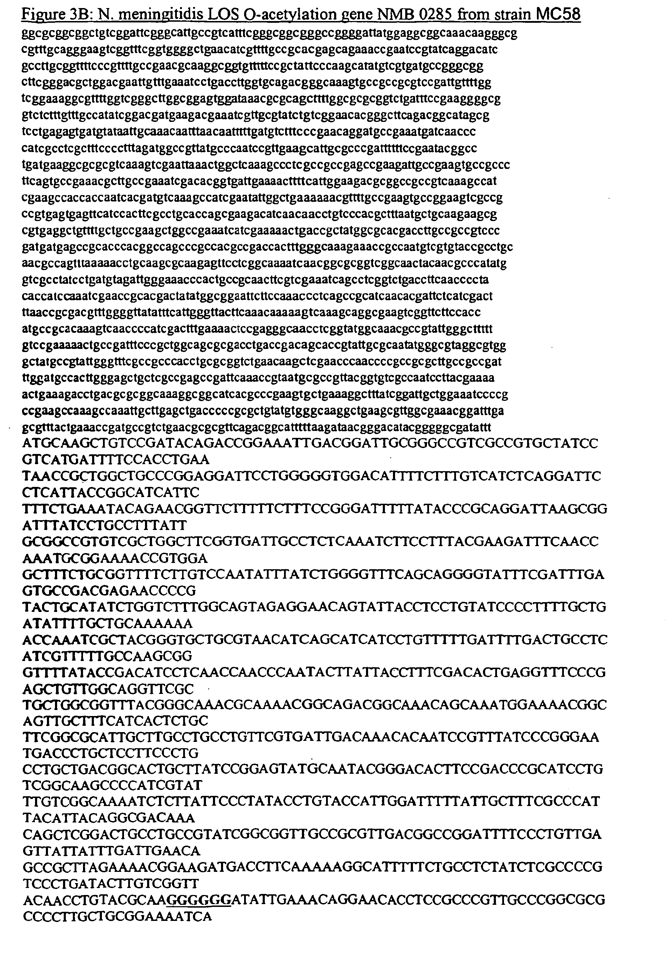

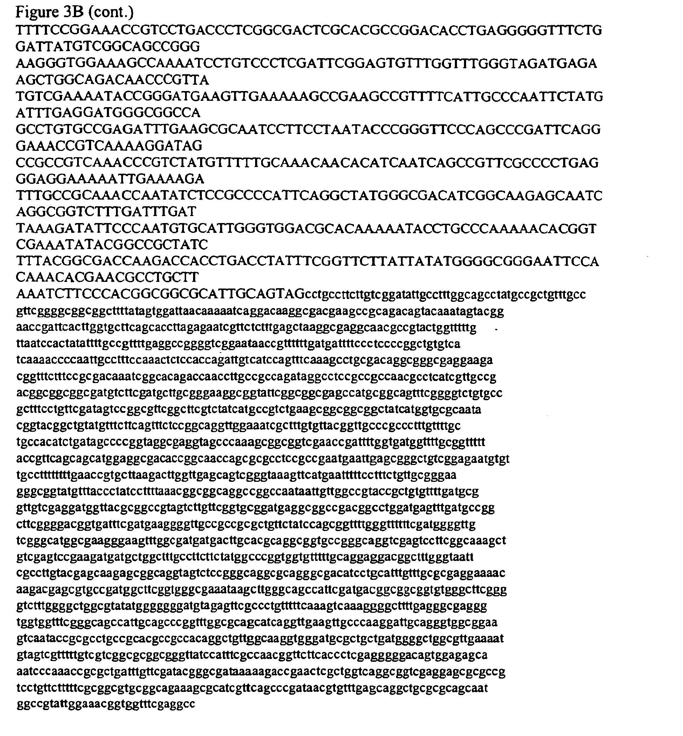

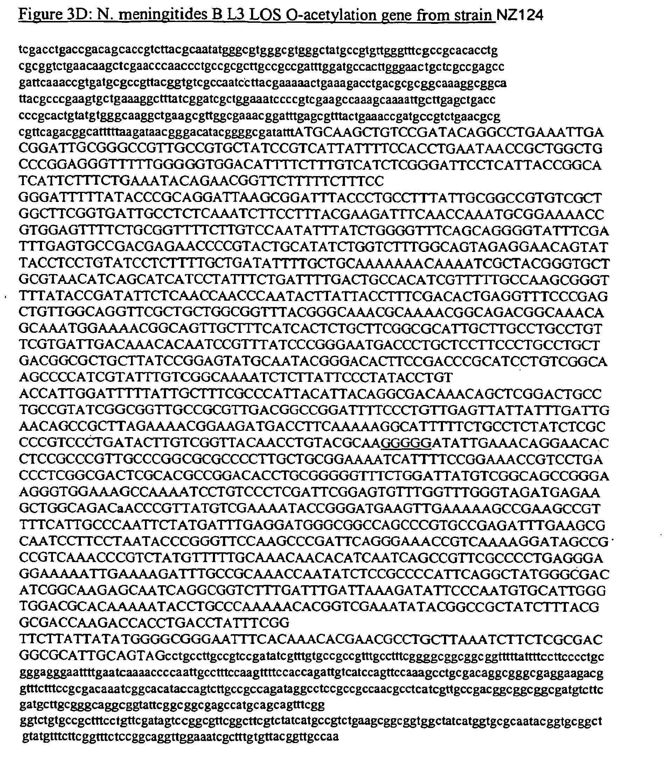

Figure 3B shows the *N. meningitidis* LOS O-acetylation gene NMB 0285 (oac1) from strain MC58 (100% identical with the strain H44/76 open reading frame gene sequence). Open reading frame in upper case, surrounding sequences (e.g. promoter sequence) in lower case. Note sequence of 6 Gs in the open reading frame (underlined) renders the open reading frame out of frame. In Figure 3A the equivalent gene NMA 2202 (oac1) from strain Z2491 is shown. Note sequence of 5 Gs in the open reading frame (upper case) renders the open reading frame in frame.
1136-GGG GGG ATA TTG AA-1150 MC58
1136-GGG GGA TAT TGA A-1149 Z2491 (NMA 2202)

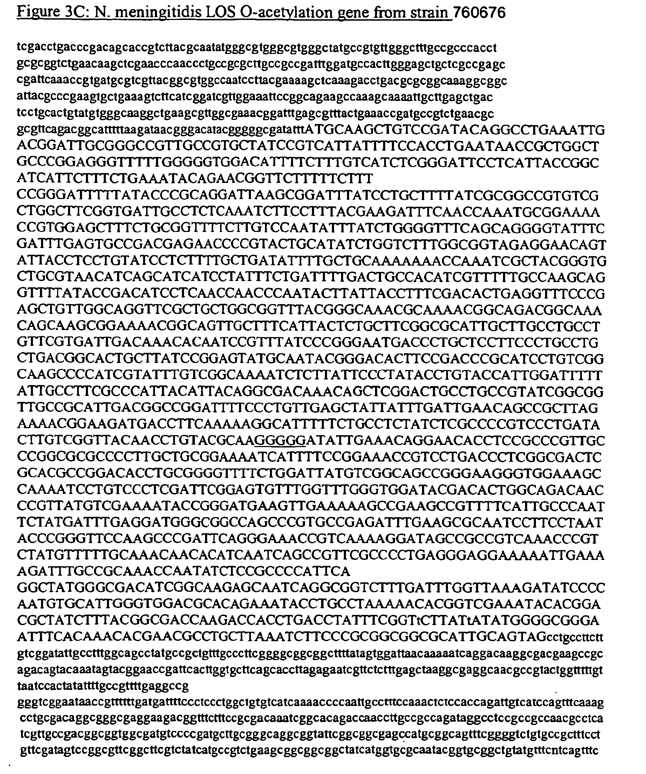

It is also in frame for strain 760676 (Figure 3C) [97% identity between open reading frame gene sequence of H44/76 and 760676 oac1).

In strain W3193 the LOS was not acetylated even though it had 5G in the region described above. This was found to be due to an additional poly G tract in the gene starting at nucleotide 354 of the ORF. 4 Gs are present in the gene at this position for strains MC58, 760676 and NZ124 (in frame, active gene), and 3 Gs were found in W3193 (out of frame, explaining its de-O-acetylated state).

### Inactivation of O-Acetylation gene in NZ124

The equivalent NMB0285 gene (oac1) was knocked-out in NZ124, a L3 strain that is O-acetylated (with oac1 in an active configuration) and is more resistant to serum induced by H44/76 (non acetylated)-derived blebs. Inactivation of the O-acetylase gene was first confirmed by mass spectroscopy analysis and the KO mutant will be used in SBA analysis to investigate further the involvement of LOS O-acetylation in the vaccine cross protection.
- NMB0285 NZ124 locus was sequenced (Figure 4D) and shared 98.6% identity with the equivalent MC58 NMB0285 locus (confirmation of a "IN PHASE" G number in NZ124). After the stop, there is an additional sequence similar to IS1106, also present in MC58 and 760676, that is absent in NZ124.
- An NMB0285 KO plasmid was constructed (pMG-T-easy vector) containing NZ124 recombinant 5' and 3' region corresponding to the 5' and 3' flanking regions of the O-Acetylation gene. KanR marker was introduced in replacement of the NMB0285 gene. This plasmid was named **pRIT 15574.**
- NZ124 oac1 KO strain was constructed - LOS derived therefrom was de-O-acetylated - further showing that oac1 is the Nmen LOS O-acetylase.

### O-Acetylation gene ON in new L3 derived strains

Blebs derived from strain H44/76 are of L3 immunotype (non-acetylated). As the blebs have reduced capacity to induced bactericidal antibodies against L3 O-acetylated strains (e.g. NZ124, BZ10) it is proposed to reintroduce a functional O-acetylation gene (oac1) into the bleb production strain derived from H44/76.

### Strategy:

- Replacement of the H44/76 NMB0285 gene (gene off) by the NZ124 gene (O-ac ON strain).
- NZ124 promoter and upstream regulation sequences was included (the NZ124 orf and the NZ124 448 upstream bp was inserted).
- Insertion took place using an Erythromycin resistance gene in sense orientation
- The importance of bleb L3 LOS O-Acetylation on vaccine heterologous protection efficacy was tested (to observe whether there was improved killing of NZ124 by H44/76 blebs modified in the above way). A combination of this L3 bleb with blebs from an O-acetylated L2 strain 760676 will also be tested to look for heterologous bactericidal antibody production.

### Results

- Above carried out and resulting LOS from modified H44/76 strain was shown to be O-acetylated at HepII by mass spectroscopy.

### Example 4: Characterization of the LOS of strains 6275 and C11 by the Ouchterlony method (immunotyping), MS/MS analysis and molecular biology analysis

### Summary

- Strains 6725 and C11 were immunotyped by immunodiffusion using specific polyclonal antibodies (Ouchterlony method). Their inner core LOS composition was determined by MS/MS analysis. Genes encoding the enzymes involved in LOS inner core decoration were analysed through PCR and sequencing.
- Based on MS/MS analysis, strain 6275 possesses two PEA residues on the Hep II. This strain was immunotyped as an L3 strain, though conventional L3 strains have only one PEA (at position 3 on HepII).
- Two different compositions of the inner core LOS of strain C11 were observed by MS-MS. One population contains one PEA residue on the Hep II while the second population contains two PEA residues. The strain was immunotyped as an L3 strain with also a very weak reaction with anti-L2 sera.

### Introduction

The inner core LOS composition of *Neisseria meningitidis* LOS appears to be more complex than previously described. Until recently, the inner core LOS was proposed to contain either no PEA or only one PEA residue on the Hep II at position 3 or 6(7). But a new inner core LOS with two PEA residues at position 3 and at position 6 (or 7) was recently described. Because this new LOS structure was described from a strain previously immunotyped as L3, this new LOS structure has been named L3v for L3 variant.

Before the discovery of the L3v structure, epidemiological data based on immunotyping of LOS have shown that around 70% of invasive serogroup B strains were L3 (PEA at position 3) and most of the remaining strains were L2 (PEA at position 6).

Based on bactericidal data obtained with a panel of L3 and L2 strains and sera from animals immunized either with L3 derived blebs or L2 derived blebs we have concluded that only anti-L3 derived sera are able to mediate the complement killing of L3 strains while only anti-L2 sera are able to kill L2 strains. But interestingly bactericidal data obtained on two new L3v strains are not in line with our previous conclusions. Indeed, the complement killing of these two strains is mediated by anti-L2 derived sera but not anti-L3 sera. These two "atypical" strains are the serogroup B strain 6275 and the serogroup C reference strain C11.

In order to understand the divergent results between immunotype and bactericidal results, the inner core composition of strains 6275 and C11 was determined by MS/MS. In addition, immunotyping of these strains was performed using the Ouchterlony method (immunodiffusion using specific polyclonal sera). The presence of functional *lpt3, lpt6* and *lgtG* genes were also analysed using molecular biological methods. These genes encode for enzymes responsible for addition on Hep II of a PEA at position 3, a PEA at position 6 and a Glucose at position 3, respectively.

### Results

### 1. Inner core composition by MS/MS analysis

The MS/MS analyses (see Figure 4) show that:
- In both strains, the α-chain LOS is the typical LNnT tetrasaccharide described for L2 and L3 strains with a terminal sialic acid group.
- The inner core LOS of strain 6275 possesses two PEA residues most probably at positions 3 and 6(7) but to be confirmed by NMR analysis.
- The C11 strain was composed of two different inner cores:
   ■ one population with one PEA (its position on Hep II is not defined, but a weak signal for a glycine on Hep II is observed which excludes a PEA at position 7)
   ■ a second population with two PEA (most probably in position 3 and 6 because a glycine was also detected on this Hep II)
- For both strains no glucose is detected on Hep II

### 2. LOS Immunotyping

The immunotyping was performed using a panel of specific antisera. Only the results obtained with the L3 antiserum and L2 antiserum are described below because the results generated with other antisera (L1, L4, L5...) were negative for strains 6275 and C11. In these experiments, strains 6275 and C11 currently used at GSK Bio (GSK6275-1&2 and GSK C11) were compared to similar strains conserved in a freezer for more than 20 years at the Amsterdam University (strains Zol 6275 and RIV C11).

The two strains 6275 (Zol 6275 and GSK6275-1&2) have similar behaviour in the immunotyping assay. They react strongly with the anti-L3 serum but not with the anti-L2 serum.

The GSK C11 strain and the RIV C11 strain also display identical results. Both strains are positive with the anti-L3 serum and weakly positive with the anti-L2 serum.

In order to confirm the very weak precipitation obtained with C11 strains and the anti-L2 serum, new immunodiffusion experiments were done in combination with 5 patients' isolates previously typed as L3,2 in 1980. The C11 results show again very weak precipitations with the anti L2 serum while the typing of the 5 disease strains is confirmed.

| Strains | Serogroup | typed 1980 | typed 2006 |
|---|---|---|---|
| 2991 | B | L3,(2) | L3,2 |
| 3072 | W | L3,2 | L3,2 |
| 3146 | C | L3,(2) | L3,(2) |
| 3151 | W | L3,2 | L3,2 |
| 3356 | B | L3,2 | L3,2 |
| GSK C11 | C | | L3,(2) |
| RIV C11 | C | | L3,(2) |

### 3. Molecular characterization

The enzyme adding the PEA in position 3 has been identified and is encoded by the gene *lpt3.* A PEA in position 3 on Hep II is detected in 70% of hypervirulent *N. meningitidis* strains and Wright and coworkers detected the *lpt3* gene by PCR in 86% of analyzed strains. This gene is not regulated by phase variation and was found to be partly deleted in various serogroup C and A strains. The addition of a PEA in position 3 has been hypothesized to be in competition with the addition of glucose at the same position. The enzyme involved is encoded by the *lgtG* gene (see WO04/015099), regulated by phase variation with a polyC tract in the ORF. The hypothesis from the Moxon laboratory is that, if the *lgtG* ORF is present and in frame, a functional enzyme is produced, glucose is added at position 3 and a PEA is not added at that position.

The gene coding for the enzyme adding a PEA in position 6 is *lpt6,* located beside the *lgtG* gene. This gene does not contain regions susceptible to be regulated by phase variation and was detected in 48% of *N.m.* (Wright et al, 2004). The gene coding for the enzyme adding a Glycine in position 7 on Hep II is not known.

MenB strain 6275 and menC strain C11 were analysed in parallel with corresponding L3 and L2 reference strains. PCR amplification and sequencing experiments were performed:
- While the presence of a full copy of *lpt3* gene was assessed (PCR), investigations were pursued on the functionality of the *lgtG* gene (presence by PCR and sequence of the polyC stretch in the ORF)
- the presence of the *lpt6* gene was assessed by PCR. If a full copy of the gene is present, we will postulate that the strain contains LOS with a PEA in position 6.

### PCR and sequence analysis of lpt3, lpt6 and IgtG genes

| | *lpt3* | *lgtG* | | *lpt6* |
|---|---|---|---|---|
| | Presence | Presence | Functionality | Presence |
| H44/76 (L3) | + | + | - | - |
| NZ124 (L3) | + | - | - | - |
| B16B6 (L2) | + | + | + | + |
| 760676(L2) | + | + | + | + |
| 6275 | + | + | +* | + |
| C11 | + | + | +* | + |

Based on those data, menB 6275 and menC C11 strains seem to be related to the L2 immunotype. However, MS/MS analysis shows for both strains two PEA groups and no Glucose. The * indicates that the lgtG gene has 14 rather than 11 (the normal number seen in active genes) consecutive C nucleotides in the phase variable region. Although this means the open reading frame is in frame and thus may produce a functional protein, it is also possible that the addition of an additional Proline residue might disrupt the protein structure and thus its function.

### 4. Summary

The next table summarises the characterization of different meningococcal strains using different methods

| Strains | Immunotyping | SBA killing | | MS/MS | | Molecular characterization | | |
|---|---|---|---|---|---|---|---|---|
| | | Anti-TrL3 | Anti-TrL2 | PEA | Glc | *lpt3* | *lpt6* | *lgtG* functional |
| H44/76 | L3 | + | - | 1 | - | + | - | - |
| NZ124 | L3 | + | - | 1 | - | + | - | - |
| 760676 | L2 | - | + | 1 | + | + | + | + |
| B16B6 | L2 | - | + | 1 | + | + | + | + |
| 6275 | L3 | - | + | 2 | - | + | + | +* |
| C11 | L3(2) | - | + | 1&2 | - | + | + | +* |

### Discussions

The diversity of the inner core LOS composition is more complex than previously described. Initially, strains without or with one PEA group on Hep II (either on position 3 or 6/7) were depicted but recently strains with two PEA groups were described. Such strains are for example the serogroup B strain 6275 and the serogroup C strain C11.

Surprisingly, immunotyping results and serum bactericidal results obtained with strains 6275 and C11 are not in line. Indeed, these two strains are typed as L3 strains but their killing is mediated by anti-L2 derived blebs sera and not by anti-L3 derived blebs sera (see next example).

A question was raised about the relevance of these strains and whether the presence of two PEA groups could be a laboratory artefact due to successive *in-vitro* culture passages. We have had the opportunity to compare different seeds of strains 6275 and C11. For each strain a seed currently used by the inventors was compared to an older seed stored for more than 20 years (at the Amsterdam University). For each strain, the two seeds have shown the same behaviour in the Ouchterlony assay suggesting absence of drift at least during these last 20 years. Nevertheless, the inner core LOS composition of the oldest seeds should be determined to confirm the presence of two PEA residues.

According to the literature around 70% of invasive meningococcal strains are L3. The Ouchterlony results obtained with L3v strains suggested that this method does not differentiate between L3 and L3v strains. Therefore, the number of "true L3" strains could be over-estimated in both studies. The *lpt3* and *lpt6* genes are responsible for the addition of PEA group at the position 3 and 6 on Hep II respectively. Around 36% of circulating strains contain both genes, 50% possess *lpt3* only and 12% possess *lpt6* only (Wright JC *et al,* 2004). Therefore potentially 36% of strains could be L3v even if typed as L3. However, recent epidemiological data obtained with a panel of MAbs specific for different inner core LOS structures suggested that less than 2% of strains have two PEA groups on Hep II (Gidney MAJ et al, Infect Imnun. 2004 72: 559-69). The difference between these two studies (36% versus 2%) could be explained by the higher sensitivity to human complement of strains possessing a PEA group on position 6 (Ram S et al J Biol Chem. 2003 278:50853-62). Taken together all the data suggest that the majority of invasive strains are "true L3".

It was suggested that *lgtG* and *lpt-3* compete for the O-3 position of Hep II, with a described bias for the addition of Glc residue over PEA residue (Wright JC *et al* 2004). This hypothesis may not be a universal rule as demonstrated by the presence of 2 PEA residues in the inner core LOS of strains 6275 and C11 even in the presence of an functional *lgtG* gene (unless the in frame lgtG gene with 14 C nucleotides in the phase variable region is not active - see above). In addition to the Moxon laboratory hypothesis another system involved in the regulation/composition of LOS inner-core structure of strain NmB was recently described. This system is the MisR/MisS two component regulatory system (Tzeng YL et al J Biol Chem. 2004 279:35053-62). In conclusion, the mechanisms involved in the composition of the inner core LOS appear to be multiple and not fully elucidated.

Five strains isolated from patients displayed a surprising LOS immunotype because they show a predominant precipitation with anti-L3 sera but also a weaker precipitation with anti-L2 serum. This is also the case with the strain C11 even if the precipitation with anti-L2 serum is very weak. The MS/MS analysis of the inner core LOS of strain C11 shows also two different inner-core compositions partially in agreement with the co-expression of L3 and L2 LOS. The L3 LOS identified by immunoprecipitation could actually be a L3v LOS (with 2 PEA residues) whilst the L2 LOS should be related to inner core LOS possessing one PEA at position 6 (to be confirmed by NMR analysis) even in absence of detectable Glc which should be present on some LOS molecule due to the detection of an apparently functional *lgtG* gene in strain C11. Nevertheless, analysis (MS/MS, molecular characterisation and SBA) of the inner core LOS of one or more of those patients isolates should be done to confirm that these L3,2 strains co-express L3v and L2 LOS.

In conclusion, strains possessing two PEA groups on their inner core are immunotyped as L3 strains but this immunotyping is not in agreement with biological reactivity in SBA and analysis of the presence of "functional" *lgtG, lpt3* and *lpt6* genes. Our data indicate that those L3v strains are probably not derived from L3 strains (due to the presence of the *lpt6* gene) and thus should be renamed.

### Example 5: Neisseria meningitidis vaccine: bivalent composition of LOS-rich bleb based vaccines

### Bleb production strains used (immunotype, genetic modifications, etc.)

| Strain | Based on: | LOS | *siaD -* | *porA -* | *msbB -* | *lgtB -* | *lst -* | *frpB -* | TrHsf up |
|---|---|---|---|---|---|---|---|---|---|
| B1854 | H44/76 | TrL3 | X | X | X | X | | X | X |
| B1948 | H44/76 | L7 | X | X | X | | X | X | X |
| B1900 | 760676 | TrL2 | X | X | X | X | | | |
| B1987 | 760676 | TrL2 | X | X | X | X | | X | |
| B1971 | 760676 | L2 | X | X | X | | | X | |
| B1984 | 760676 | L2 | X | X | X | | X | X | |

### Production of blebs from culture done with or without desferal

- B1854, B1948, B1971, B1984 and B1987 blebs were produced from cultures in the presence of desferal
- B 1900 blebs were obtained from a culture without desferal

### Methods

Animal procedure: Groups of 30 mice were immunized three times with OMV (containing around 15-20% of detoxified LOS) by the intramuscular route (IM) on day 0, 21 and 28. Each inoculation was made up of 5µg (protein content) of non-adsorbed OMVs. The OMVs were produced from *Neisseria meningitidis* (Nmen) strains engineered so that capsular polysaccharides and PorA were down regulated and LOS detoxified (*msbB*) mutation). The production strains (see table above) were derived from either genetically modified wild type strain H44/76 (in this case they expressed either the L7 LOS or the TrL3 LOS) or genetically modified wild type strain 760676 (in this case they expressed the L2 LOS, sialylated or not, or the TrL2 LOS). Blebs were isolated from strains grown in culture conditions described above. On day 42, blood samples were taken for analysis by serum bactericidal assay (SBA) using a panel of *Neisseria meningitidis* strains (see table 1). SBA's were performed either on pooled blood samples or on individual sera (10 to 30 sera per group).

Infant rat experiments were performed as followed. Groups of 20 seven days old rats were immunized by the IM route on day (0, 14, 28 and 63). Each inoculation was made up of 10µg (protein content) of non-adsorbed blebs. Blood samples were taken 14 days after the fourth injection.

Guinea-pig experiments were performed as followed. Groups of 20 guinea-pigs were immunized by the IM route on day (0, 14, 28). Each inoculation was made up of 20µg (protein content) of non-adsorbed blebs. Blood samples were taken 14 days after the third injection.

Rabbit experiments were performed as followed. Groups of 5 New Zealand white rabbits were immunized by the IM route on day (0, 21, 42). Each inoculation was made up of 20µg (protein content) of non-adsorbed blebs. Blood samples were taken 14 days after the third injection.

SBA's using liquid culture with desferal: *N. meningitidis* strains were cultivated overnight on MH + 1% Polyvitex + 1% horse serum Petri Dishes at 37°C + 5% CO₂. They were sub-cultured for 3 hours in a liquid TSB medium supplemented with 50µM of desferal (iron chelator) at 37°C under shaking to reach an OD of approximately 0.5 at 470 nm. Serum samples were inactivated for 40 min at 56°C and then diluted 1/10 or 1/50 in PBS-glucose 0.1% and then twofold diluted (8 dilutions) in a volume of 25 µl in flat bottom microplates. Bacteria were diluted in PBS-glucose 0.1% to yield 5300 CFU/ml and 18.8µl of this dilution was added to the serum dilution. Rabbit complement (6.2µl) was also added to each well. After 75 min of incubation at 37°C under shaking, 50µl of MH + 0.9% agar are added to the wells and 50µl of PBS+ 0.9% agar approximately 30 min later. The microplates are incubated overnight at 37°C +CO₂. The CFU's are counted and the percentage of killing is calculated. The SBA titer is the dilution giving 50% of killing.

SBA's using agar culture (without desferal): *N. meningitidis* strains were cultivated overnight on BHI + 1% horse serum Petri Dishes at 37°C + 5% CO₂. They were sub-cultured for 4 hours BHI + 1% horse serum at 37°C + 5% CO₂. Serum samples were inactivated for 40 min at 56°C and then diluted 1/10 in PBS-glucose 0.1% and then twofold diluted (8 dilutions) in a volume of 25 µl in flat bottom microplates. Bacteria were diluted in PBS-glucose 0.1% to yield 6400 CFU/ml and 12.5µl of this dilution was added to the serum dilution. Rabbit complement (12.µl) was also added to each well. After 75 min of incubation at 37°C under shaking, 50µl of TSB + 0.9% agar are added to the wells and 50µl of PBS+ 0.9% agar approximately 30 min later. The microplates are incubated overnight at 35 or 37°C +CO₂. The CFU's are counted and the percentage of killing is calculated. The SBA titer is the dilution giving 50% of killing.

### Inner-core LOS compositions of different immunotypes

- L3 = one PEA on HepII (most probably on position 3) and no additional Acetyl on inner core GlcNAc
- "L3" = one PEA on HepII (most probably on position 3) and one additional Acetyl on inner core GlcNAc
- L2 = one PEA on HepII (most probably on position 6) and one additional Acetyl on inner core GlcNAc
- Variant = two PEA's on HepII (most probably on positions 3 and 6) and one additional Acetyl on inner core GlcNAc ; though note in strain W3193 no OAc on HepII was present
- L10 and L11 = structures not determined
- L3, "L3", L2 and variant harbor the LNnT tetrasaccharide (sialylated or not).

### Results

The results summarized in Table 1 below and Figure 5 show that
• L2 derived blebs induce bactericidal antibodies against NmenB L2 strains but not against NmenB L3 strains (excepted for the strain M97250687) while L3 derived blebs induce a protective response against NmenB L3 and "L3" strains, against the serogroup A strain 3125 (L10) and the serogroup Y strain M01.0240539 (M01.539 in the table) but not against L2 strains. The structure of the 3125 L10 LOS by mass spectroscopy is shown in Figure 6.
• In addition, L2 derived blebs induce protection against strains harboring a variant LOS (NmenB 6275 and NmenC C11 strains) and also against others strains from serogroup C (C19), serogroup Y (S1975), serogroup A (F8238, L11) and serogroup W-135 (strains S4383 and 3193).
• A bivalent vaccine based on enriched LOS blebs derived from L2 and L3 strains induce a protective response (bactericidal antibodies) against all tested strains (whatever the serogroup and the LOS immunotype) (a bivalent vaccine with L2 NS and TrL3 OMVs must be tested).
• Non-truncated L2 LOS induce better "cross-protection" than *IgtB* mutants (=TrL2). This is not the case for L3 derived blebs (TrL3=L7).
• Non-sialylated L2 blebs induce similar level of bactericidal antibodies than sialylated-L2 blebs (at least against the strain 6275).
• According to the targeted strains in SBA, interferences are observed or not with the bivalent vaccines indicating that a fine tuning of the bivalent formulation/composition is requested to ensure the best efficacy (SBA titers) against all the tested strains).
• Decoration of inner core has a major impact on the induction of cross-bactericidal antibodies.

**Table 1. Cross-bactericidal antibodies induced by monovalent and bivalent LOS enriched OMV vaccines. SBA titers (for 50% killing) and seroconversion (%).**

| **Strains** | **Group** | **LOS** | **Vaccines (non-adsorbed)** | | | | | | |
|---|---|---|---|---|---|---|---|---|---|
| | | | **L7(B1948)** | **L2(B1971)** | **L7+L2** | **TrL3(B1854)** | **TrL2(B1900)** | **TrL3+TrL2** | **Buffer** |
| | | | | | | | | | |
| H44/76 | B | L3 | 4979 | <100 | 5267 | 5507 | <100 | 12754 | <100 |
| M97250687 | B | L3 | 10123 | 3800 | 5739 | >51200 | 469 | >51200 | <100 |
| NZ124 | B | "L3" | 479 | <20 | 228 | 949 | <20 | 523 | <20 |
| | | | | | | | | | |
| 760676 | B | L2 | <20 | 329 | 213 | <20 | 104 | 40 | <20 |
| 2986 | B | L2 | <200 | 5288 | 2670 | <200 | 1486 | 1486 | <200 |
| B16B6 | B | L2 | <100 | 1746 | 1401 | <100 | 2236 | 1097 | <100 |
| | | | | | | | | | |
| 6275 | B | Variant | <20 | 267 | 43 | <20 | 28 | <20 | <20 |
| | | | 0% | 100% | 60% | 0% | 60% | 5% | 0% |
| 6275* | B | Variant | <100 | 2748 | 1456 | <100 | 657 | 456 | <100 |
| | | | | | | | | | |
| C11 | C | Variant | <20 | 2767 | 1303 | <20 | 405 | 176 | <20 |
| | | | 0% | 100% | 100% | 0% | 100% | 100% | 0% |
| S1975 | Y | Variant | <20 | >5120 | >5120 | <20 | >5120 | 2205 | <20 |
| | | | 0% | 100% | 100% | 20% | 100% | 100% | 10% |
| M01.539 | Y | ?? | 235 | 18 | 140 | 168 | 19 | 76 | <20 |
| | | | 100% | 20% | 75% | 89% | 20% | 70% | 20% |
| F8238 | A | L11 | l42 | >5120 | >5120 | 42 | 3469 | 1665 | <20 |
| | | | 100% | 100% | 100% | 60% | 100% | 100% | 10% |
| 3125 | A | L10 | 82 | 14 | NT | 207 | <20 | NT | <20 |
| | | | 88% | 20% | | 100% | 0% | | 0% |
| S4383 | W | "L3" | 54 | 1259 | NT | NT | NT | NT | NT |
| | | | 55% | 100% | | | | | |
| 3193 | W | Variant | <20 | >5120 | NT | NT | NT | NT | NT |
| | | | 0% | 100% | | | | | |

| | | | | | | | | | |
|---|---|---|---|---|---|---|---|---|---|
| H44/76, M97250687, NZ124, 760676, 2986 and B16B6: SBA on pooled sera. Other strams: SBA on individual sera. Non-serogroup B strains and L2 strains : SBA done from agar cultures without desferal (unlike exceptions) ^{$} Mean of two assays using liquid culture + desferal * Pooled sera tested using agar culture (without desferal) | | | | | | | | | |

The results in Table 2 show that
• The cross-protection conferred by L2 derived blebs (L2 with or without terminal sialic acid and TrL2) against non-serogroup B strains and strain 6275 is also observed in guinea-pigs, rabbits and infant rats.
• In general, L2 derived blebs are not able to induce significant level of bactericidal Abs able to mediate the complement killing of L3 strains excepted strain M97250687 (M687 in the table) (data on strain H44/76 using infant rats and rabbit sera are not reliable and must be repeated due to background of activities measured on negative sera).
• In guinea-pigs, TrL2 blebs seem to induce higher level of bactericidal antibodies than L2 *lst-* and L2 blebs while in infants rats, mice and rabbits the following ranking is observed: L2 ≥ L2 *lst-* ≥ TrL2.
• Those results show that sialylation of L2 LOS is not required to induce the induction of bactericidal antibodies and that TrL2 blebs (*lgtB* mutation) is also able to elicit the production of bactericidal antibodies (as TrL3 LOS).

**Table 2. Cross-bactericidal antibodies induced by monovalent LOS enriched L2 dervied OMVs. SBA titers (for 50% killining) and seroconversion (%).**

| | | MenB | | | | | | | MenA | MenC | MenY | MenW |
|---|---|---|---|---|---|---|---|---|---|---|---|---|
| | | 760676 | L2 B16B6 | 2986 | H44/76 | L3 M687 | NZ124 | Variant 6275 | L11 F8238 | Variant C11 | Variant S1975 | "L3" S4383 |
| Infant rats | | | | | | | | | | | | |
| TrL2 (B1987) | PI V | 463 | 129 | 1447 | *410* | 122 | 50 | 1822 | 1250 | 143 | 6446 | 6010 |
| L2lst-(B1984) | PI V | 1812 | 486 | 4396 | *2188* | 138 | 50 | 385 | 3090 | 365 | 8923 | 1577 |
| L2 Ist+ (B1971) | PI V | 1519 | 996 | 5294 | *110* | 168 | *983 ?* | 961 | 3604 | 554 | 11000 | 1478 |
| Ctrl(-) | PI V | 50 | 50 | 50 | *360* | 50 | 50 | 50 | 50 | 50 | 50 | 154 |

| Mice | | | | | | | | | | | | |
|---|---|---|---|---|---|---|---|---|---|---|---|---|
| TrL2 (B1987) | PIII | 186 | 1692 | 6081 | 50 | 107 | 10 | 410 | 1929 | 1131 | 2661 | 2782 |
| L2 Ist- (B1984) | PIII | 232 | 1645 | 7557 | 50 | 321 | 10 | 748 | 3929 | 1804 | 8032 | 1371 |
| L2 Ist+ (B1971) | PIII | 790 | 1330 | 8216 | 50 | 582 | 10 | 1305 | 5082 | 2525 | 11731 | 1040 |
| Ctri(-) | PIII | 50 | 50 | 50 | 50 | 50 | 10 | 50 | 50 | 50 | 50 | 50 |

| Guinea pigs | | | | | | | | | | | | |
|---|---|---|---|---|---|---|---|---|---|---|---|---|
| TrL2 (B1987) | PIII | >12800 | 8129 | 4194 | 50 | 182 | 50 | 7253 | 3952 | 627 | 10207 | 7200 |
| L2 lst-(B1984) | PIII | >12800 | 5473 | 6608 | 594 | 770 | *123 ?* | 7326 | 5431 | 1491 | >12800 | 5417 |
| L2 lst+ (B1971) | PIII | >12800 | 5945 | 6484 | 457 | 527 | 50 | 4148 | 5061 | 1051 | 5967 | 5036 |
| Ctrt(-) | PIII | 50 | 50 | 50 | 50 | 50 | 50 | 50 | 124 | 50 | 665 | 50 |

| Rabbits | | | | | | | | | | | | |
|---|---|---|---|---|---|---|---|---|---|---|---|---|
| TrL2 (B1987) | Pré | 59 | 50 | 50 | *562* | 50 | 50 | 50 | 50 | 50 | 50 | 50 |
| | PIII | 752 | 362 | 346 | *134* | 50 | 50 | 157 | 221 | 66 | 636 | 723 |
| L2 lst- (B 1984) | Pré | 63 | 50 | 50 | *318* | 50 | 50 | 50 | 50 | 50 | 50 | 50 |
| | PIII | 1541 | 507 | 430 | *307* | 72 | 50 | 317 | 414 | 122 | 1386 | 1238 |
| L2 Ist+ (B1971) | Pré | 50 | 50 | 50 | *178* | 50 | 50 | 50 | 50 | 50 | 50 | 62 |
| | PIII | 4727 | 1540 | 551 | *116* | 90 | 50 | 750 | 892 | 224 | 2419 | 3667 |
| Ctrl(-) | Pré | 50 | 50 | 50 | *73* | 50 | 50 | 50 | 50 | 50 | 50 | 173 |
| | PIII | 50 | 50 | 50 | *236* | 50 | 50 | 50 | 50 | 50 | 50 | 372 |

| | | | | | | | | | | | | |
|---|---|---|---|---|---|---|---|---|---|---|---|---|
| SBA's were done on pooled sera excepted for rabbit sera which were tested individually | | | | | | | | | | | | |

**Conclusion**: A combination of L2 and L3 based LOS vaccines can elicit antibodies that can kill Men A, B, C, W135 and Y strains - the first time this has been shown. L2-based vaccines can kill L2, variant (L3v) strains and L11 strains. L3-based vaccines can kill L3, "L3" (less well), and L10 strains. Although truncated (lgtB(-)) and full-length (or lst(-)) alpha chains can kill, truncated seems beneficial for L3 vaccines and full length (or Ist(-)) seems beneficial for L2 vaccines. A combination of these two LOS-based vaccines shows much potential as a vaccine against *N. meningitidis.*

### Example 6: Neisseria meningitidis vaccine: bivalent OMVs (L7 derived and L2 derived) induce cross-protection against ABCWY strains

### Methods

### Animal procedures:

- Groups of 30 mice were immunized three times with bivalent OMVs formulation (L3 derived OMV + L2 derived OMV containing around 15-20% of detoxified LOS) by the intramuscular (IM) route on day 0, 21 and 28. Each inoculation was made up of 0.8µg+0.8µg (LOS content) of non-adsorbed OMVs. The OMVs were produced from *Neisseria meningitidis* (Nmen) strains engineered so that capsular polysaccharides and PorA were down regulated and LOS detoxified (*msbB* mutation). The production strains were derived from either genetically modified wild type strain H44/76 (L3) or genetically modified wild type strain 760676 (L2) (see below a table describing the genetic modifications and culture conditions). On day 42, blood samples were taken for analysis by serum bactericidal assay (SBA) using a panel of 22 *Neisseria meningitidis* strains from serogroups A, B, C, W135 (W) and Y. SBA's were performed on pooled blood samples. Sera were from experiments 20060425 and 20060426.
- Infant rat experiment was performed as followed. Seven days old rats (n=20 per group) were immunized by the IM route on day (0, 14, 28 and 63). Each inoculation was made up of 1.6µg+1.6µg (LOS content) of non-adsorbed OMVs. Blood samples were taken 14 days after the fourth injection and pooled (experiment 20060484).
- Guinea-pig experiment was performed as followed. Groups of 20 guinea-pigs were immunized by the IM route on day (0, 14, 28). Each inoculation was made up of 3.2µg+3.2µg (LOS content) of non-adsorbed OMVs. Blood samples were taken 14 days after the third injection and pooled (experiment 20060487).
- Rabbit experiment was performed as followed. Groups of 5 New Zealand with rabbits were immunized by the IM route on day (0, 21, 42). Each inoculation was made up of 3.2µg+3.2µg (LOS content) of non-adsorbed OMVs. Blood samples were taken before the first injection and 14 days after the third injection and they were tested individually in SBA (experiment 20060486).

### OMVs production strains (immunotype) and genetic modifications

| | | *siaD -* | *porA -* | *msbB -* | *lgtB -* | *lst*- | *frpB -* | TrHsfup | NspA up |
|---|---|---|---|---|---|---|---|---|---|
| B1854 | TrL3 | X | X | X | X | | X | X | |
| B1948 | L7 | X | X | X | | X | X | X | |
| B2084 | TrL2 | X | X | X | X | | | | X |
| B2071 | NSL2 | X | X | X | | X | | | X |

Production of OMVs from culture done with or without desferal

B1854 and B1948 OMVs were produced from cultures done with desferal

B2071 and B2084 OMVs were obtained form a culture done without desferal

SBA: *N. meningitidis* strains were cultivated overnight on Petri Dishes at 37°C + 5% CO₂. They were sub-cultured for 4 hours on Petri Dishes without or with desferal (iron chelator) 37°C + 5% CO₂. Serum samples were inactivated for 40 min at 56°C and then diluted 1/10 or 1/50 in PBS-glucose 0.1% and then twofold diluted in a volume of 25 µl in flat bottom microplates. Then 25 µl of a mix of bacteria (diluted in PBS-glucose 0.1% to yield -100-150 CFU per well) and baby rabbit complement (final concentration m microwell: 12.5% v/v) was added to the serum dilution. After 75 min of incubation at 37°C under shaking, 2 layers of agar (0.9%) were added to the wells. The microplates were incubated overnight at 35 or 37°C +CO₂. The CFU's were counted and the percentage of killing was calculated. The SBA titer is the dilution giving 50% of killing.

### Inner-core LOS compositions of different immunotypes

- L3 = one PEA on HepII (most probably on position 3) and no additional Acetyl on inner core GlcNAc
- "L3" = one PEA on HepII (most probably on position 3) and one additional Acetyl on inner core GlcNAc
- L2 = one PEA on HepII (most probably on position 6) and one additional Acetyl on inner core GlcNAc
- Variant = two PEA's on HepII (most probably on positions 3 and 6) and in general one additional Acetyl on inner core GlcNAc
- L10 and L11 = structures not fully determined
- L3, "L3 ", L2 and variant harbor the LNnT tetrasaccharide (sialylated or not).

### Results

Based on a four fold increase of bactericidal titers (using pooled serum samples from control animal or pre vaccine serum sample as comparator) the results (see table on following page) show that
- Non-adsorbed bivalent formulations containing either L7 + NSL2 OMVs or TrL3+TrL2 OMVs are able to confer a cross-protection against *N. meningitidis* strains belonged to serogroups A, B, C, W and Y.
- This cross-protection is observed not only in mice but also in infant rats, guinea-pigs and rabbits.
- Nevertheless these formulations are not able to elicit a protective response against strains expressing the L4 LOS immunotype. In these experiments, sera are not bactericidal against a L10 strain.
- Most of the strains expressing a L3 or "L3" or variant or L2 LOS are killed by mouse, infant rat and guinea pig sera. Against those strains, the percentage of cross-protection is close to 90%
- The cross-protection appears to be lower in rabbits.

### Conclusion

A bivalent vaccine based on OMV derived from L3 and L2 *N. meningitidis* strains is able to confer protection against *N. meningitidis* strains expressing either a L3 or "L3" or variant (L3v) or L2 LOS. This protection is not restricted to a given serogroup.

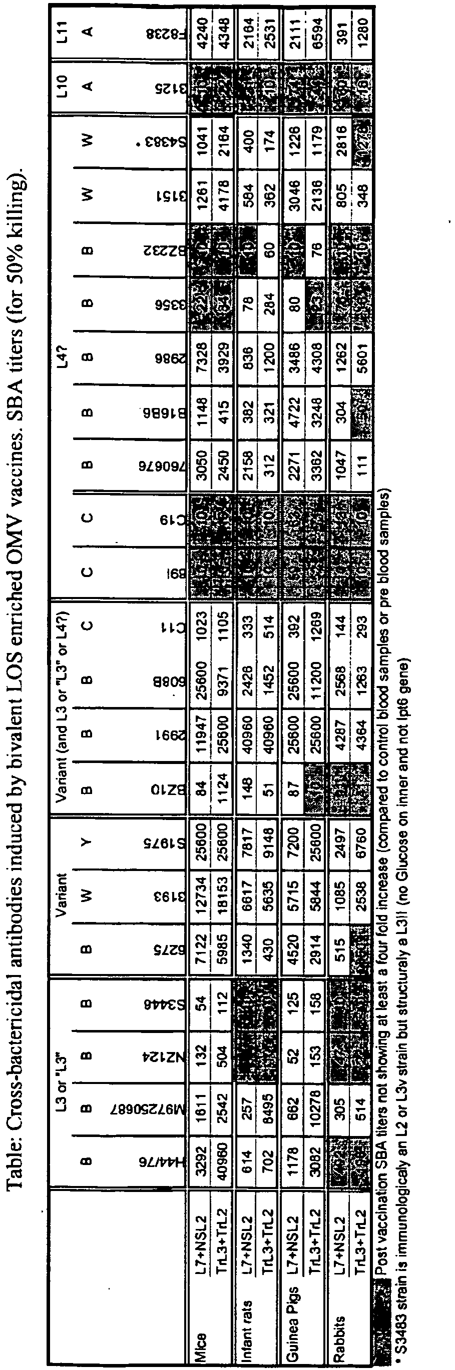

### Example 7: Impact of OAc on immunogenicity of L7 OMVs

### Methods

### Animal procedures:

- Groups of 30 mice were immunized three times with OMV (containing around 15-20% of detoxified LOS) by the intramuscular (1M) route on day 0, 21 and 28. Each inoculation was made up of 0.8pg (LOS content) of non-adsorbed OMVs. The OMVs were produced from *N. meningitidis* strains engineered so that capsular polysaccharides and PorA were down regulated and LOS detoxified (*msbB* mutation). The production strains were denved from genetically modified wild type strain H44/76 (see below a table describing the genetic modifications and culture conditions). On day 42, blood samples were taken for analysis by serum bactericidal assay (SBA) using *N. meningitidis* strains. SBA's were performed on individual sera. Sera were from experiment 20060634.
- Guinea-pig experiment was performed as followed. Groups of 20 guinea-pigs were immunized by the IM route on day (0, 14, 28). Each inoculation was made up of 3.2 µg (LOS content) of non-adsorbed OMVs. Blood samples were taken 14 days after the third injection (experiment 20060636).

### OMVs production strains (immunotype) and genetic modifications

| | | *siaD -* | *porA -* | *msbB -* | *Ist -* | *frpB -* | TrHsfup | *nmb0285* |
|---|---|---|---|---|---|---|---|---|
| B1948 | L7 OAc- | X | X | X | X | X | X | OFF |
| B2103 | L7 OAc+ | X | X | X | X | X | X | ON |

| | | | | | | | | |
|---|---|---|---|---|---|---|---|---|
| B1948 and B2103 OMVs were produced from cultures grown with desferal | | | | | | | | |

SBA: *N. meningitidis* strains (H44/76 and M97250687 which are OAc- and NZ124 which is OAc+) were cultivated overnight on Petri Dishes at 37°C + 5% CO₂. They were sub-cultured for 4 hours on Petri Dishes with desferal (iron chelator) 37°C + 5% CO₂. Serum samples were inactivated for 40 min at 56°C and then diluted 1/10 or 1/50 in PBS-glucose 0.1% and then twofold diluted in a volume of 25 µl in flat bottom microplates. Then 25µl of a mix of bacteria (diluted in PBS-glucose 0.1% to yield ~100-150 CFU per well) and baby rabbit complement (final concentration in micro-well: 12.5% v/v) was added to the serum dilutions. After 75 min of incubation at 37°C under shaking, 2 layers of agar (0.9%) were added to the wells. The microplates were incubated overnight at 35 or 37°C +CO₂. The CFU's were counted and the percentage of killing was calculated. The SBA titer is the dilution giving 50% of killing.

### Results

In mice L7 OAc+ OMVs are more immunogenic than L7 OAc- OMVs (see following table). Indeed sera from mice immunized with.L7 OAc+ OMVs show higher complement mediated bactericidal activity than sera from mice immunized with L7 OAc- OMVs. This is observed against OAc- and OAc+ wild type strains. Nevertheless, significant differences are only observed against OAc- strains (H44/76 and M97205687).

**Table: Bactericidal antibodies induced by L7 OAc- OMVs (B 1948) and L7 OAc+ OMVs (B2103) in mice. GMT (for 50% killing) and confidence intervals at 95%.**

| | B1948 (OAc-) OMVs | B2103 (OAC+) OMVs |
|---|---|---|
| H44/76 | 3699 (1987-6886) | 9122* (6381-13041) |
| M97.250687 | 2768 (1741-4401) | 7780* (5594-10820) |
| NZ124 | 117 (61-225) | 191 (105-348) |

| | | |
|---|---|---|
| **p*<0.05 compared to GMT for B1948 immunized mice | | |

In guinea pigs, both types of OMVs show in SBA similar immunogenicity against OAc+ and OAc- wild type strains (see table below).

**Table. Bactericidal antibodies induced by L7 OAc- OMVs (B 1948) and L7 OAc+ OMVs (B2103) in guinea pigs. GMT (for 50% killing) and confidence intervals at 95%.**

| | B1948 (OAc-) OMVs | B2103 (OAC+) OMVs |
|---|---|---|
| H44/76 | 1283 (896-1836) | 1392 (876-2212) |
| NZ124 | 69 (38-128) | 43 (25-74) |

### Conclusion

O-acetylation of inner-core GlcNAc has demonstrated to increase the immunogenicity (SBA titer) of OMVs in mice but not guinea pig.

## Claims

1. A vaccine comprising L3V LOS and L3 LOS from Neisserial strains, wherein the L3V and the L3 LOS has the following structure: 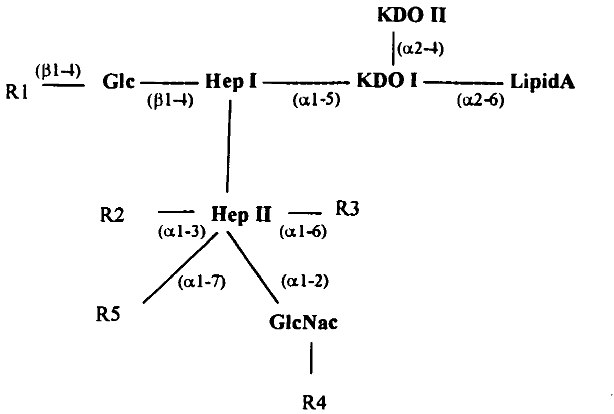 and wherein for the L3V LOS: 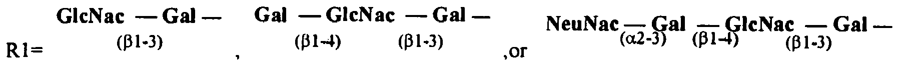 R2= PEA,
R3= PEA,
R4= H or OAc, R5=H, PEA, or Gly,
and wherein for the L3 LOS: 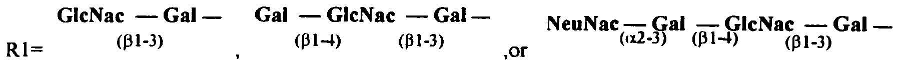 R2= PEA,
R3= H,
R4= H or OAc,
R5=H or Gly
further comprising a pharmaceutically acceptable excipient.

2. The vaccine of claim 1 further comprising L10 LOS from a Neisserial strain, optionally isolated from a *Neisseria meniningitidis* A, B, C, W 135 or Y strain.

3. The vaccine of claim 1 or 2, wherein the composition further comprises L4 LOS of the following structure: 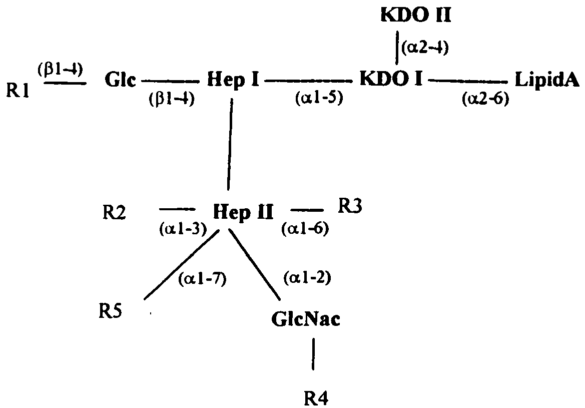 wherein for the L4 LOS: 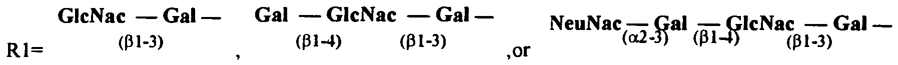 R2= H,
R3= PEA,
R4= OAc,
R5=Gly.

4. The vaccine of claim 3, wherein the L4 LOS has R4 = H instead of OAc and/or has R5 = H instead of Gly.

5. The vaccine of claims 1-4, wherein the L3V LOS is isolated from a *Neisseria meningitidis* A, B, C, W135 or Y strain.

6. The vaccine of claims 1-5, wherein the L3 LOS is isolated from a *Neisseria meningitidis* A, B, C, W 135 or Y strain.

7. The vaccine of claims 1-6, wherein the L3V LOS, L3 LOS, L10 LOS, L4 LOS, L3V and L3 LOS, L3V and L10 LOS, L3 and L10 LOS, L4 and L3V LOS, L4 and L3 LOS, L4 and L10 LOS, L3V and L3 and L10 LOS, L3V and L3 and L4 LOS, L3V and L4 and L10 LOS, L3 and L4 and L10 LOS, or L3V and L3 and L10 and L4 LOS is conjugated to a protein carrier.

8. The vaccine of claims 1-7, wherein the L3V LOS, L3 LOS, L10 LOS, L4 LOS, L3V and L3 LOS, L3V and L10 LOS, L3 and L10 LOS, L4 and L3V LOS, L4 and L3 LOS, L4 and L10 LOS, L3V and L3 and L10 LOS, L3V and L3 and L4 LOS, L3V and L4 and L10 LOS, L3 and L4 and L10 LOS, or L3 V and L3 and L10 and L4 LOS has a detoxified lipid A moiety, for instance lacking a secondary acyl chain consistent with the LOS having been isolated from a msbB(-) neisserial strain.

9. The vaccine of claims 1-8, wherein the L3V LOS, L3 LOS, L10 LOS, L4 LOS, L3V and L3 LOS, L3V and L10 LOS, L3 and L10 LOS, L4 and L3V LOS, L4 and L3 LOS, L4 and L10 LOS, L3V and L3 and L10 LOS, L3V and L3 and L4 LOS, L3 V and L4 and L10 LOS, L3 and L4 and L10 LOS, or L3 V and L3 and L10 and L4 LOS is present in the immunogenic composition as a purified LOS preparation.

10. The vaccine of claims 1-9, wherein the L3V LOS, L3 LOS, L10 LOS, L4 LOS, L3V and L3 LOS, L3 V and L10 LOS, L3 and L10 LOS, L4 and L3V LOS, L4 and L3 LOS, L4 and L10 LOS, L3V and L3 and L10 LOS, L3V and L3 and L4 LOS, L3 V and L4 and L10 LOS, L3 and L4 and L10 LOS, or L3 V and L3 and L10 and L4 LOS is present in the immunogenic composition as a liposomal preparation.

11. The vaccine of claims 1-10, wherein the L3V LOS, L3 LOS, L10 LOS, L4 LOS, L3 V and L3 LOS, L3 V and L10 LOS, L3 and L10 LOS, L4 and L3 V LOS, L4 and L3 LOS, L4 and L10 LOS, L3 V and L3 and L10 LOS, L3V and L3 and L4 LOS, L3 V and L4 and L10 LOS, L3 and L4 and L10 LOS, or L3 V and L3 and L10 and L4 LOS is present in the immunogenic composition as a bleb preparation.

12. A vaccine composition comprising an effective amount of the vaccine of claims 1-II, one or more conjugated capsular polysaccharides or oligosaccharides derived from the following strains: meningococcus serogroup A, meningococcus serogroup C, meningococcus serogroup W-135, meningococcus serogroup Y, and *H. influenzae* type b, and a pharmaceutically acceptable carrier.

13. A use of the vaccine of claims 1-11 or the vaccine of claim 12 in the manufacture of a medicament for the prevention or treatment of disease caused by one or more *N. meningitidis* serogroups selected from the following list: A, B, C, W135, and Y.

14. A vaccine of claims 1-12 for use in the prevention or treatment of disease caused by one or more *N.meningitidis* serogroups selected from the following list: A, B, C, W135, and Y.

15. A process of manufacturing the vaccine of claims 1-12 comprising the step of isolating the L3V LOS and the L3 LOS, combining the L3V and L3 LOS, and formulating the L3 V and L3 LOS with a pharmaceutically acceptable excipient.

16. An immunogenic composition comprising L3V LOS from a Neisserial strain for use in the prevention or treatment of *N. meningitidis* immunotype L2 disease, or an immunogenic composition comprising L2 LOS from a Neisserial strain for use in the prevention or treatment of *N. meningitidis* immunotype L3 V disease wherein the L3 V LOS has the following structure: 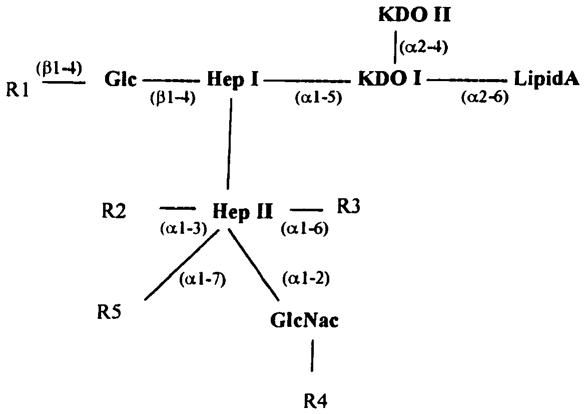 wherein: 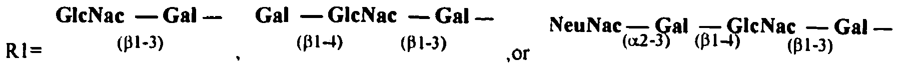 R2= PEA,
R3= PEA,
R4= H or OAc,
R5=H, PEA, or Gly.

17. The immunogenic composition of claim 16, wherein the L2 LOS has the following structure: 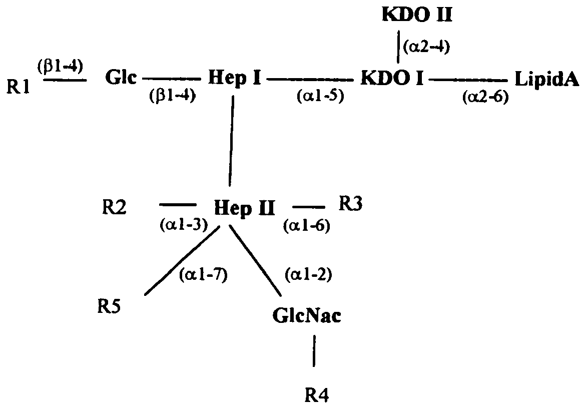 wherein: 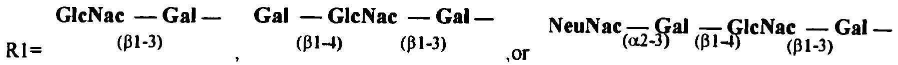 R2= Glc,
R3= PEA,
R4= H or OAc,
R5=H, PEA, or Gly.

## Patentansprüche

1. Impfstoff, umfassend L3V LOS und L3 LOS aus Neisseria-Stämmen, wobei das L3V und das L3 LOS die folgende Struktur haben: 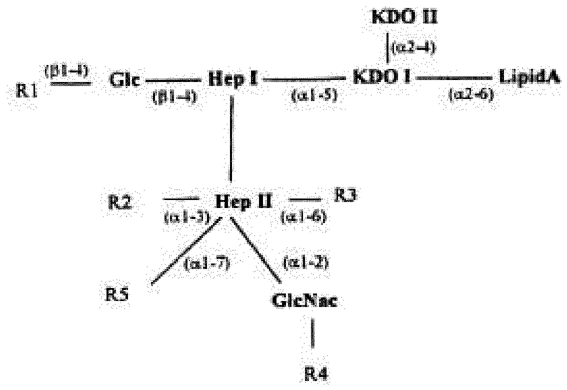 und wobei für das L3V LOS: 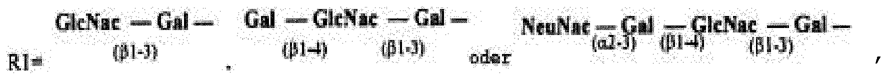 R2 = PEA,
R3 = PEA,
R4 = H oder OAc,
R5 = H, PEA oder Gly, und wobei für das L3 LOS: 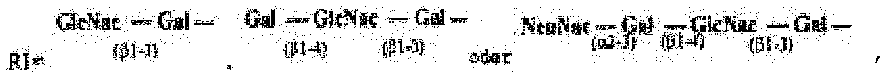 R2 = PEA, R3 = H,
R4 = H oder OAc,
R5 = H oder Gly,
ferner umfassend einen pharmazeutisch akzeptablen Exzipienten.

2. Impfstoff gemäß Anspruch 1, ferner umfassend L10 LOS aus einem Neisseria-Stamm, gegebenenfalls isoliert aus einem Neisseria meningitidis A-, B-, C-, W135- oder Y-Stamm.

3. Impfstoff gemäß Anspruch 1 oder 2, wobei die Zusammensetzung ferner L4 LOS der folgenden Struktur umfasst: 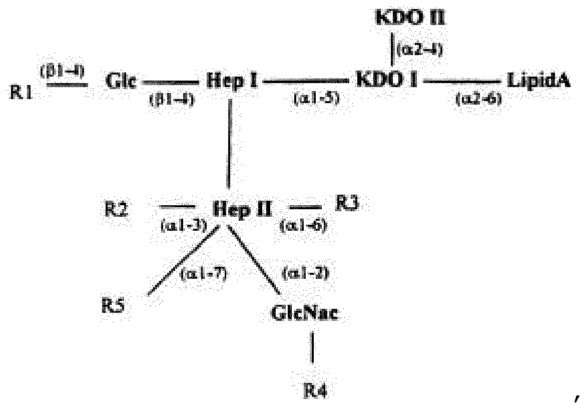 wobei für das L4 LOS: 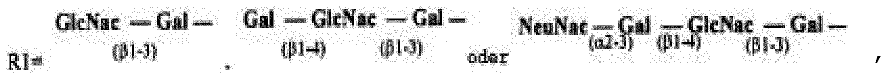 R2 = H,
R3 = PEA,
R4 = OAc,
R5 = Gly.

4. Impfstoff gemäß Anspruch 3, wobei das L4 LOS R4 = H anstatt OAc aufweist und/oder R5 = H anstatt Gly aufweist.

5. Impfstoff gemäß den Ansprüchen 1 bis 4, wobei das L3V LOS aus einem Neisseria meningitidis A-, B-, C-, W135-oder Y-Stamm isoliert ist.

6. Impfstoff gemäß den Ansprüchen 1 bis 5, wobei das L3 LOS aus einem Neisseria meningitidis A-, B-, C-, W135- oder Y-Stamm isoliert ist.

7. Impfstoff gemäß den Ansprüchen 1 bis 6, wobei das L3V LOS, L3 LOS, L10 LOS, L4 LOS, L3V und L3 LOS, L3V und L10 LOS, L3 und L10 LOS, L4 und L3V LOS, L4 und L3 LOS, L4 und L10 LOS, L3V und L3 und L10 LOS, L3V und L3 und L4 LOS, L3V und L4 und L10 LOS, L3 und L4 und L10 LOS oder L3V und L3 und L10 und L4 LOS an einen Proteinträger konjugiert ist.

8. Impfstoff gemäß den Ansprüchen 1 bis 7, wobei das L3V LOS, L3 LOS, L10 LOS, L4 LOS, L3V und L3 LOS, L3V und L10 LOS, L3 und L10 LOS, L4 und L3V LOS, L4 und L3 LOS, L4 und L10 LOS, L3V und L3 und L10 LOS, L3V und L3 und L4 LOS, L3V und L4 und L10 LOS, L3 und L4 und L10 LOS, oder L3V und L3 und L10 und L4 LOS eine detoxifizierte Lipid A-Gruppe aufweist, der z.B. eine sekundäre Acylkette fehlt, übereinstimmend mit dem LOS, das aus einem msbB(-) Neisseria-Stamm isoliert wurde.

9. Impfstoff gemäß den Ansprüchen 1 bis 8, wobei das L3V LOS, L3 LOS, L10 LOS, L4 LOS, L3V und L3 LOS, L3V und L10 LOS, L3 und L10 LOS, L4 und L3V LOS, L4 und L3 LOS, L4 und L10 LOS, L3V und L3 und L10 LOS, L3V und L3 und L4 LOS, L3V und L4 und L10 LOS, L3 und L4 und L10 LOS, oder L3V und L3 und L10 und L4 LOS in der immunogenen Zusammensetzung als eine gereinigte LOS-Zubereitung vorhanden ist.

10. Impfstoff gemäß den Ansprüchen 1 bis 9, wobei das L3V LOS, L3 LOS, L10 LOS, L4 LOS, L3V und L3 LOS, L3V und L10 LOS, L3 und L10 LOS, L4 und L3V LOS, L4 und L3 LOS, L4 und L10 LOS, L3V und L3 und L10 LOS, L3V und L3 und L4 LOS, L3V und L4 und L10 LOS, L3 und L4 und L10 LOS, oder L3V und L3 und L10 und L4 LOS in der immunogenen Zusammensetzung als eine liposomale Zubereitung vorhanden ist.

11. Impfstoff gemäß den Ansprüchen 1 bis 10, wobei das L3V LOS, L3 LOS, L10 LOS, L4 LOS, L3V und L3 LOS, L3V und L10 LOS, L3 und L10 LOS, L4 und L3V LOS, L4 und L3 LOS, L4 und L10 LOS, L3V und L3 und L10 LOS, L3V und L3 und L4 LOS, L3V und L4 und L10 LOS, L3 und L4 und L10 LOS, oder L3V und L3 und L10 und L4 LOS in der immunogenen Zusammensetzung als eine Bläschen-Zubereitung vorhanden ist.

12. Impfstoff-Zusammensetzung, umfassend eine wirksame Menge des Impfstoffs gemäß den Ansprüchen 1 bis 11, ein oder mehrere konjugierte Kapselpolysaccharide oder -oligosacccharide, abgeleitet aus den folgenden Stämmen: Meningococcus Serogruppe A, Meningococcus Serogruppe C, Meningococcus Serogruppe W-135, Meningococcus Serogruppe Y und H. influenzae Typ b, und einen pharmazeutisch akzeptablen Träger.

13. Verwendung des Impfstoffs gemäß den Ansprüchen 1 bis 11 oder des Impfstoffs gemäß Anspruch 12 in der Herstellung eines Medikaments zur Prävention oder Behandlung von Krankheit, die durch eine oder mehrere N. meningitidis-Serogruppen verursacht wird, die aus der folgenden Liste ausgewählt sind: A, B, C, W135 und Y.

14. Impfstoff gemäß den Ansprüchen 1 bis 12 zur Verwendung in der Prävention oder Behandlung von Krankheit, die durch eine oder mehrere N. meningitidis-Serogruppen verursacht wird, die aus der folgenden Liste ausgewählt sind: A, B, C, W135 und Y.

15. Verfahren zur Herstellung des Impfstoffs gemäß den Ansprüchen 1 bis 12, umfassend den Schritt des Isolierens des L3V LOS und des L3 LOS, das Kombinieren des L3V und des L3 LOS, und das Formulieren des L3V und L3 LOS mit einem pharmazeutisch akzeptablen Exzipienten.

16. Immunogene Zusammensetzung, umfassend L3V LOS aus einem Neisseria-Stamm, zur Verwendung in der Prävention oder Behandlung von N. meningitidis-Immunotyp L2-Krankheit, oder immunogene Zusammensetzung, umfassend L2 LOS aus einem Neisseria-Stamm, zur Verwendung in der Prävention oder Behandlung von N. meningitidis-Immunotyp L3V-Krankheit, wobei das L3V LOS die folgende Struktur hat: 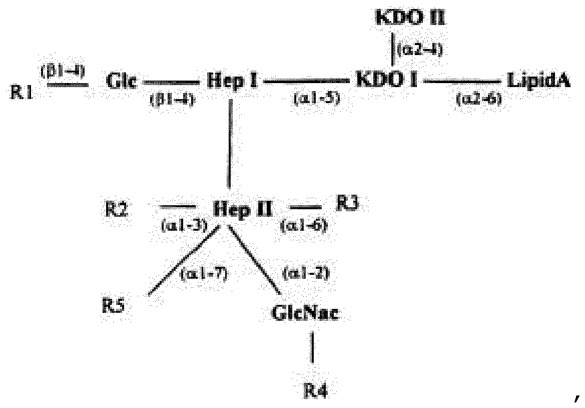 wobei: 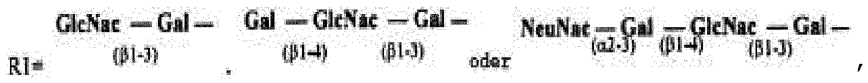 R2 = PEA,
R3 = PEA,
R4 = H oder OAc,
R5 = H, PEA oder Gly.

17. Immunogene Zusammensetzung gemäß Anspruch 16, wobei das L2 LOS die folgende Struktur hat: 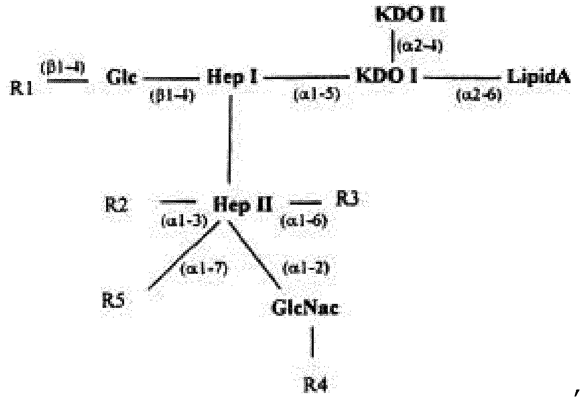 wobei: 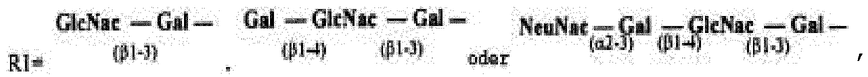 R2 = Glc,
R3 = PEA,
R4 = H oder OAc,
R5 = H, PEA oder Gly.

## Revendications

1. Vaccin comprenant L3V LOS et L3 LOS des souches de *Neisseria,* dans lequel les L3V et L3 LOS ont la structure suivants : 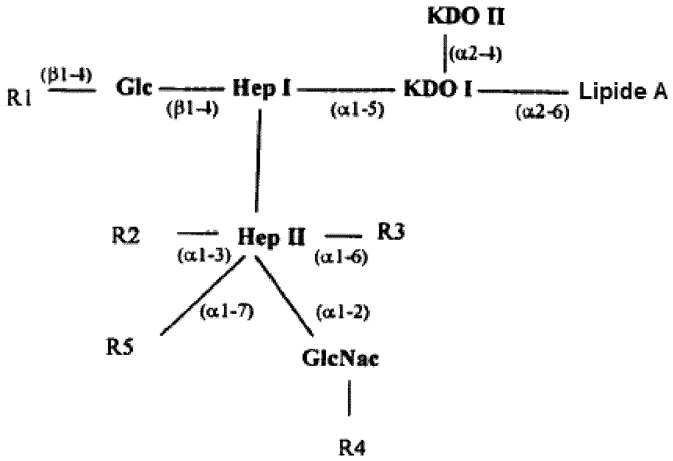 et dans laquelle pour L3V LOS :
R1 = 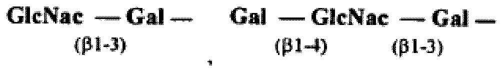 ou 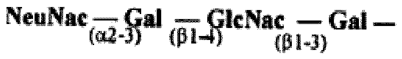
R2 = PEA,
R3 = PEA,
R4 = H ou OAc,
R5 = H, PEA ou Gly,
et dans laquelle pour L3 LOS :
R1 = 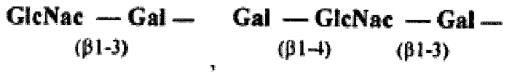 ou 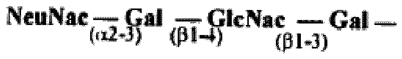
R2 = PEA,
R3 = H,
R4 = H ou OAc,
R5 = H ou Gly
comprenant en outre un excipient pharmaceutiquement acceptable.

2. Vaccin selon la revendication 1, comprenant en outre L10 LOS d'une souche de *Neisseria,* éventuellement isolée d'une souche A, B, C, W135 ou Y de *Neisseria meningitidis.*

3. Vaccin selon la revendication 1 ou 2, dans lequel la composition comprend en outre L4 LOS de structure suivants : 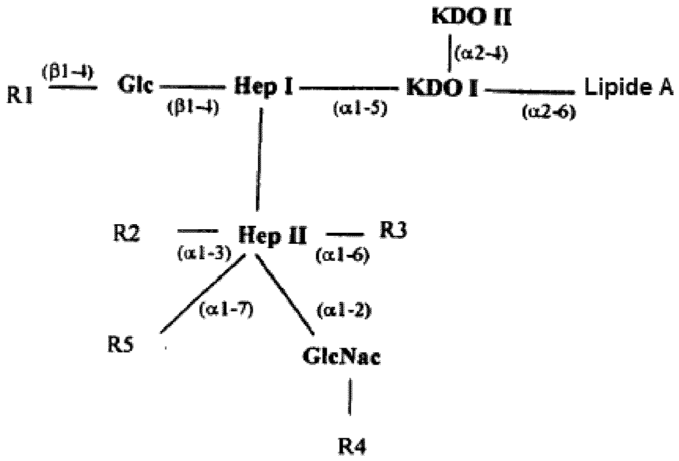 dans laquelle pour L4 LOS :
R1 = 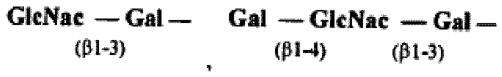 ou 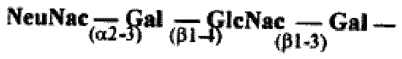 R2 = H,
R3 = PEA,
R4 = OAc,
R5 = Gly.

4. Vaccin selon la revendication 3, dans lequel L4 LOS a R4 = H au lieu de OAc et/ou a R5 = H au lieu de Gly.

5. Vaccin selon les revendications 1 à 4, dans lequel L3V LOS est isolé d'une souche A, B, C, W135 ou Y de *Neisseria meningitidis.*

6. Vaccin selon les revendications 1 à 5, dans lequel L3 LOS est isolé d'une souche A, B, C, W135 ou Y de *Neisseria meningitidis.*

7. Vaccin selon les revendications 1 à 6, dans lequel les L3V LOS, L3 LOS, L10 LOS, L4 LOS, L3V et L3 LOS, L3V et L10 LOS, L3 et L10 LOS, L4 et L3V Los, L4 et L3 LOS, L4 et L10 LOS, L3V et L3 et L10 LOS, L3V et L3 et L4 LOS, L3V et L4 et L10 LOS, L3 et L4 et L10 LOS ou L3V et L3 et L10 et L4 LOS sont conjugués dans un véhicule protéique.

8. Vaccin selon les revendications 1 à 7, dans lequel les L3V LOS, L3 LOS, L10 LOS, L4 LOS, L3V et L3 LOS, L3V et L10 LOS, L3 et L10 LOS, L4 et L3V LOS, L4 et L3 LOS, L4 et L10 LOS, L3V et L3 et L10 LOS, L3V et L3 et L4 LOS, L3V et L4 et L10 LOS, L3 et L4 et L10 LOS ou L3V et L3 et L10 et L4 LOS ont un fragment de lipide A détoxifié, par exemple, dépourvu d'une chaîne acyle secondaire conforme au LOS ayant été isolé d'une souche msbB(-) de *Neisseria.*

9. Vaccin selon les revendications 1 à 8, dans lequel les L3V LOS, L3 LOS, L10 LOS, L4 LOS, L3V et L3 LOS, L3V et L10 LOS, L3 et L10 LOS, L4 et L3V LOS, L4 et L3 LOS, L4 et L10 LOS, L3V et L3 et L10 LOS, L3V et L3 et L4 LOS, L3V et L4 et L10 LOS, L3 et L4 et L10 LOS ou L3V et L3 et L10 et L4 LOS sont présents dans la composition immunogène en tant que préparation de LOS purifiée.

10. Vaccin selon les revendications 1 à 9, dans lequel les L3V LOS, L3 LOS, L10 LOS, L4 LOS, L3V et L3 LOS, L3V et L10 LOS, L3 et L10 LOS, L4 et L3V LOS, L4 et L3 LOS, L4 et L10 LOS, L3V et L3 et L10 LOS, L3V et L3 et L4 LOS, L3V et L4 et L10 LOS, L3 et L4 et L10 LOS ou L3V et L3 et L10 et L4 LOS sont présents dans la composition immunogène en tant que préparation liposomale.

11. Vaccin selon les revendications 1 à 10, dans lequel les L3V LOS, L3 LOS, L10 LOS, L4 LOS, L3V et L3 LOS, L3V et L10 LOS, L3 et L10 LOS, L4 et L3V LOS, L4 et L3 LOS, L4 et L10 LOS, L3V et L3 et L10 LOS, L3V et L3 et L4 LOS, L3V et L4 et L10 LOS, L3 et L4 et L10 LOS ou L3V et L3 et L10 et L4 LOS sont présents dans la composition immunogène en tant que préparation vésiculaire.

12. Composition vaccinale comprenant une quantité efficace de vaccin selon les revendications 1 à 11, un ou plusieurs polysaccharides ou oligosaccharides capsulaires conjugués dérivés des souches suivantes : méningocoque de sérogroupe A, méningocoque de sérogroupe C, méningocoque de sérogroupe W-135, méningocoque de sérogroupe Y et *H. influenzae* de type b et un véhicule pharmaceutiquement acceptable.

13. Utilisation du vaccin selon les revendications 1 à 11 ou vaccin selon la revendication 12 dans la production d'un médicament pour la prévention ou le traitement d'une maladie provoquée par un ou plusieurs sérogroupes de *N*. *meningitidis* choisis dans la liste suivante : A, B, C, W135 et Y.

14. Vaccin selon les revendications 1 à 12 à utiliser dans la prévention ou le traitement d'une maladie provoquée par un ou plusieurs sérogroupes de *N*. *meningitidis* choisis dans la liste : A, B, C, W135 et Y.

15. Procédé de production du vaccin selon les revendications 1 à 12 comprenant l'étape consistant à isoler les L3V LOS et L3 LOS, combiner les L3V et L3 LOS et formuler les L3V et L3 LOS avec un excipient pharmaceutiquement acceptable.

16. Composition immunogène comprenant L3V LOS d'une souche de *Neisseria* à utiliser dans la prévention ou le traitement d'une maladie d'immunotype L2 de *N*. *meningitidis* ou composition immunogène comprenant L2 LOS d'une souche de *Neisseria* à utiliser dans la prévention ou le traitement d'une maladie d'immunotype L3V de *N*. *meningitidis,* L3V LOS ayant la structure suivante : 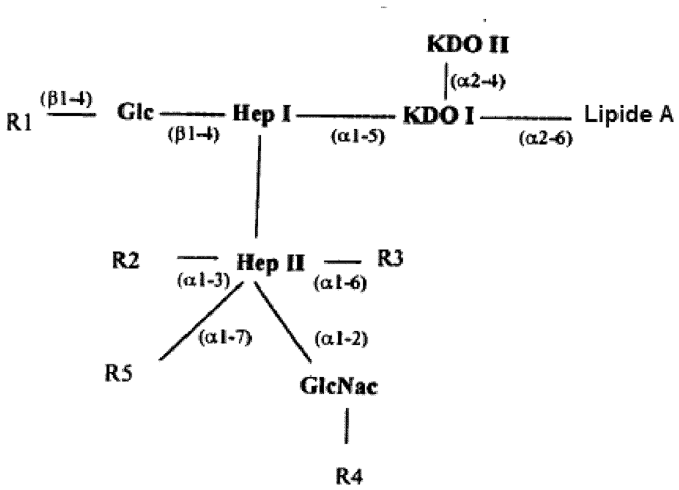 dans laquelle
R1 = 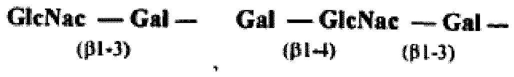 ou 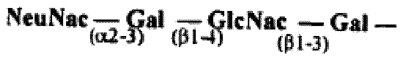 R2 = PEA,
R3 = PEA,
R4 = H ou OAc,
R5 = H, PEA ou Gly.

17. Composition immunogène selon la revendication 16, dans laquelle L2 LOS a la structure suivante : 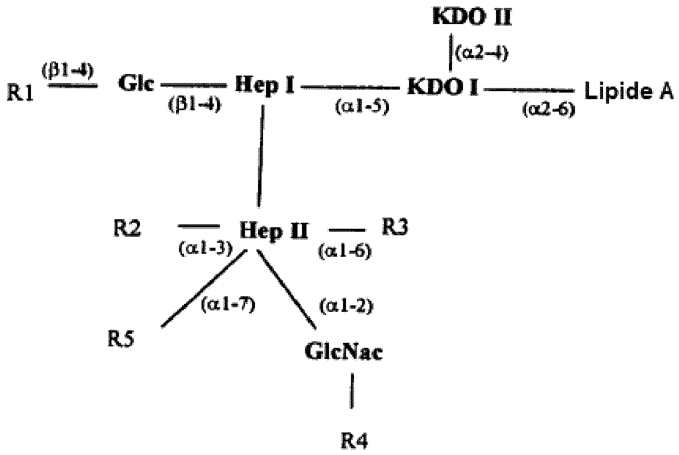 dans laquelle R1 = 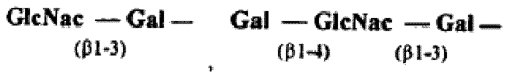 ou 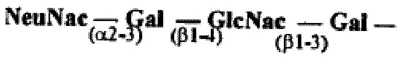 R2 = Glc,
R3 = PEA,
R4 = H ou OAc,
R5 = H, PEA ou Gly.
